(19) Europäisches Patentamt
European Patent Office
Office européen des brevets

(11) Veröffentlichungsnummer: **0 025 489**
**B1**

(12) **EUROPÄISCHE PATENTSCHRIFT**

(45) Veröffentlichungstag der Patentschrift: **20.07.83**

(51) Int. Cl.³: **C 07 D 333/64,** C 07 D 409/06, C 07 D 409/12, A 61 K 31/38

(21) Anmeldenummer: **80104136.9**

(22) Anmeldetag: **16.07.80**

(54) **Neue vinyloge Carboxamide, diese enthaltende Arzneimittel und Verfahren zur Herstellung.**

(30) Priorität: **31.07.79 DE 2931010**

(43) Veröffentlichungstag der Anmeldung: **25.03.81 Patentblatt 81/12**

(45) Bekanntmachung des Hinweises auf die Patenterteilung: **20.07.83 Patentblatt 83/29**

(84) Benannte Vertragsstaaten: **AT BE CH DE FR GB IT LI LU NL SE**

(56) Entgegenhaltungen:
DE-A-2 804 842

**Chemical Abstracts, Band 87, Nr. 13, 26. September 1977 Columbus, Ohio, USA Zh.V. Bren et al. «Basicity and structure of azomethines and their structural analogs. XVIII. Basicity and structure of 2-(N-arylaminomethylene)-3(2H)-benzo(b)thiophenones, -benzo(b)furanones and -benzo(b) selenophenones» Seite 568, Spalte 2, bis Seite 569, Spalte 1, Abstract Nr. 101741u**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Engel, Wolfhard, Dr. Dipl.-Chem., Mozartstrasse 13, D-7950 Biberach 1 (DE)**
Erfinder: **Trummlitz, Günter, Dr. Dipl.-Chem., Buchenweg 27, D-7951 Warthausen (DE)**
Erfinder: **Seeger, Ernst, Dr. Dipl.-Chem., Alpenstrasse 39, D-7950 Biberach 1 (DE)**
Erfinder: **Kähling, Joachim, Dr., Haydnweg 8, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 025 489 B1

ACTORUM AG

# Neue vinyloge Carboxamide, diese enthaltende Arzneimittel und Verfahren zur Herstellung

In der DE-A-2 804 842 wird u.a. die Verbindung 2-($\alpha$-Piperidino-methoxycarbonylmethylen)-5-methyl[2H]benzothiophen-3-on der Formel

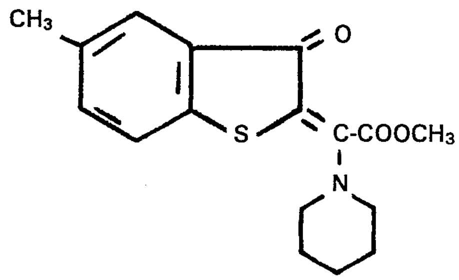

beschrieben (siehe Seite 20), welche antiallergische Eigenschaften aufweist.

Es wurde nun gefunden, dass die neuen vinylogen Carboxamide der allgemeinen Formel

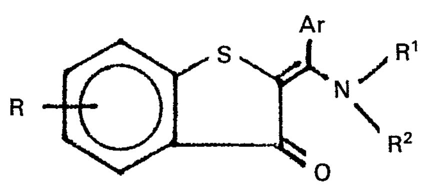
(I)

wertvolle pharmakologische Eigenschaften aufweisen, insbesondere stark antikonvulsive Wirkungen.

Gegenstand der vorliegenden Erfindung sind somit die neuen Verbindungen der allgemeinen Formel I, Verfahren zu ihrer Herstellung und diese Verbindungen enthaltende Arzneimittel.

In der obigen allgemeinen Formel I bedeuten:

Ar einen Phenylrest, der durch 1 oder 2 Halogenatome, Alkylgruppen mit 1 bis 3 Kohlenstoffatomen, die Amino-, Nitro-, Cyan- oder eine Trifluormethylgruppe substituiert sein kann, oder einen Pyridinylrest,

R ein Wasserstoff- oder Chloratom oder die Methyl- oder Methoxygruppe,

$R_1$ ein Wasserstoffatom oder die Methylgruppe,

$R_2$ ein Wasserstoffatom, einen geradkettigen oder verzweigten Alkylrest mit höchstens 6 Kohlenstoffatomen, einen 2-Propenyl- oder 2-Propinylrest, einen gegebenenfalls methylsubstituierten Cycloalkylrest mit 3 bis höchstens 8 Kohlenstoffatomen oder den Rest -A-$R_4$,

wobei

A eine Alkylengruppe mit 2 oder 3 Kohlenstoffatomen und

$R_4$ die Hydroxygruppe, der Methylamino-, Dimethylamino-, N-Methyl-ethylamino-, Diethylamino-, Pyrrolidino-, Piperidino-, Hexamethylenimino-, Morpholino- oder 4-Methyl-1-piperazinyl-Rest darstellt.

Zur Erläuterung des Erfindungsgegenstandes seien an dieser Stelle folgende Verbindungen beispielhaft genannt:

(E)-2-[(Amino)phenylmethylen]-benzo[b]thiophen-3(2H)-on
(E)-2-[(Methylamino)phenylmethylen]-benzo[b]thiophen-3(2H)-on,
(E)-2-[(Dimethylamino)phenylmethylen]-benzo[b]thiophen-3(2H)-on,
(E)-2-[(Amino)-(2-fluorphenyl)methylen]-benzo[b]thiophen-3(2H)-on,
(E)-2-[(Amino)-(2-chlorphenyl)methylen]-benzo[b]thiophen-3(2H)-on,
(E)-2-[[[2-(Dimethylamino)ethyl]amino]phenylmethylen]-benzo[b]thiophen-3(2H)-on,
(E)-2-[[(2-Hydroxyethyl)amino]phenylmethylen]-benzo[b]thiophen-3(2H)-on,
(E)-2-[(Amino)-(2-methylphenyl)-methylen]-benzo[b]thiophen-3(2H)-on,
(E)-2-[(Ethylamino)phenylmethylen]-benzo[b]thiophen-3(2H)-on,
(E)-2-[(Amino)-(2-bromphenyl)methylen]-benzo[b]thiophen-3(2H)-on,
(E)-2-[(Amino)-(2-iodphenyl)methylen]-benzo[b]thiophen-3(2H)-on,
(E)-2-[(Amino)-(2-ethylphenyl)methylen]-benzo[b]thiophen-3(2H)-on,
(E)-2-[(Amino)-(2-propylphenyl)methylen]-benzo[b]thiophen-3(2H)-on,
(E)-2-[(Amino)-(4-chlorphenyl)methylen]-benzo[b]thiophen-3(2H)-on,
(E)-2-[(Amino)-(4-methylphenyl)methylen]-benzo[b]thiophen-3(2H)-on,
(E)-2-[(Amino)-(3,4-dichlorphenyl)methylen]-benzo[b]thiophen-3(2H)-on,
(E)-2-[(Amino)-(4-fluorphenyl)methylen]-benzo[b]thiophen-3(2H)-on,
(E)-2-[(Amino)-(4-pyridinyl)methylen]-benzo[b]thiophen-3(2H)-on,
(E)-2-[(Amino)-(3-pyridinyl)methylen]-benzo[b]thiophen-3(2H)-on,
(E)-2-[(Amino)phenylmethylen]-5-chlorbenzo[b]thiophen-3(2H)-on,
(E)-2-[(Amino)phenylmethylen]-6-chlorbenzo[b]thiophen-3(2H)-on,
(E)-2-[(Amino)-(2-chlorphenyl)methylen]-5-chlorbenzo[b]thiophen-3(2H)-on,
(E)-2-[(Amino)-(2-pyridinyl)methylen]benzo[b]thiophen-3(2H)-on,
(E)-2-[(Amino)-(2-fluorphenyl)methylen]-5-chlorbenzo[b]thiophen-3(2H)-on,
(E)-2-[[(2-Propenyl)amino]phenylmethylen]-benzo[b]thiophen-3(2H)-on,
(E)-2-[[[3-(Dimethylamino)propyl]amino]phenylmethylen]-benzo[b]thiophen-3(2H)-on,
(E)-2-[[[2-(Dimethylamino)propyl]amino]phenylmethylen]-benzo[b]thiophen-3(2H)-on,
(E)-[[[3-(Dimethylamino)-2-propyl]amino]phenylmethylen]-benzo[b]thiophen-3(2H)-on,
(E)-2-[[[2-(N-Methyl-ethylamino)ethyl]amino]phenylmethylen]-benzo[b]thiophen-3(2H)-on,
(E)-2-[[[2-(Diethylamino)ethyl]amino]phenylmethylen]-benzo[b]thiophen-3(2H)-on,
(E)-2-[[[2-(1-Pyrrolidinyl)ethyl]amino]phenylmethylen]-benzo[b]thiophen-3(2H)-on,
(E)-2-[[[2-(1-Piperidinyl)ethyl]amino]phenylmethylen]-benzo[b]thiophen-3(2H)-on,

(E)-2-[[[2-(Hexahydro-1-azepinyl)ethyl]amino]phenylmethylen]-benzo[b]thiophen-3(2H)-on,
(E)-2-[[[2-(4-Morpholinyl)ethyl]amino]phenylmethylen]-benzo[b]thiophen-3(2H)-on,
(E)-2-[(Propylamino)phenylmethylen]-benzo[b]thiophen-3(2H)-on,
(E)-2-[(Butylamino)phenylmethylen]-benzo[b]thiophen-3(2H)-on,
(E)-2-[[(2-Propyl)amino]phenylmethylen]-benzo[b]thiophen-3(2H)-on,
(E)-2-[[(2-Methylpropyl)amino]phenylmethylen]-benzo[b]thiophen-3(2H)-on,
(E)-2-[[(1,1-Dimethylethyl)amino]phenylmethylen]-benzo[b]thiophen-3(2H)-on,
(E)-2-[[(3-Hydroxypropyl)amino]phenylmethylen]-benzo[b]thiophen-3(2H)-on,
(E)-2-[(Amino)-(3-methylphenyl)methylen]-benzo[b]thiophen-3(2H)-on,
(E)-2-[(Methylamino)-(2-ethylphenylmethylen]-benzo[b]thiophen-3(2H)-on,
(E)-2-[(Ethylamino)-(2-ethylphenyl)methylen]-benzo[b]thiophen-3(2H)-on,
(E)-2-[(Amino)-(2-nitrophenyl)methylen]-benzo[b]thiophen-3(2H)-on,
(E)-2-[(Amino)-(2-aminophenyl)methylen]-benzo[b]thiophen-3(2H)-on,
(E)-2-[(Amino)-(2-cyanophenyl)methylen]-benzo[b]thiophen-3(2H)-on,
(E)-2-[(Amino)phenylmethylen]-6-methylbenzo[b]thiophen-3(2H)-on,
(E)-2-[(Amino)phenylmethylen]-5-methylbenzo[b]thiophen-3(2H)-on,
und
(E)-2-[(Amino)phenylmethylen]-6-methoxybenzo[b]thiophen-3(2H)-on,

Besonders bevorzugte Verbindungen der obigen allgemeinen Formel I sind jedoch diejenigen, in denen

Ar einen Phenylrest, der gegebenenfalls in o-Stellung durch Fluor, Chlor, Brom, Methyl oder Ethyl substituiert sein kann,

R ein Wasserstoffatom,

$R_1$ ein Wasserstoffatom oder die Methylgruppe und

$R_2$ ein Wasserstoffatom, die Methyl- oder Ethylgruppe, die 2-Hydroxy-ethyl oder 2-(Dimethylamino)ethylgruppe bedeuten.

Die Verbindungen der obigen allgemeinen Formel I werden erfindungsgemäss nach folgenden Verfahren hergestellt:

1. Durch Umsetzung von 2-Acyl-benzo[b]thiophen-3-olen der allgemeinen Formel II

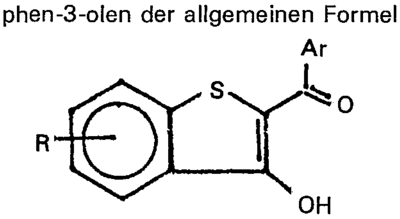

(II)

oder Enolethern der allgemeinen Formel III

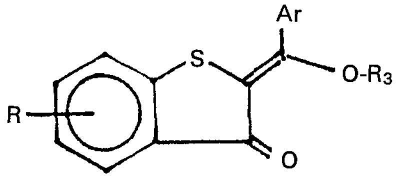

(III)

mit Aminen der allgemeinen Formel IV oder mit Ammoniumsalzen der allgemeinen Formel IVa oder mit Harnstoffen der allgemeinen Formel V:

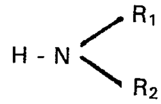

(IV)

$H_2\overset{\oplus}{N}R_1R_2 \quad \overset{\ominus}{B}$

(IVa)

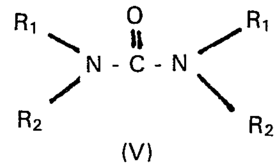

(V)

In den allgemeinen Formeln II, III, IV, IVa und V haben Ar, R, $R_1$ und $R_2$ die eingangs angegebenen Bedeutungen, $R_3$ ist ein Alkyl-, Alkenyl- oder gegebenenfalls im Benzolkern durch Halogenatome, Nitro- oder Methylgruppen substituierter Phenylalkyl-Rest mit maximal 20 Kohlenstoffatomen, $B^-$ ist das Anion einer ein- oder mehrbasischen schwachen bis mittelstarken anorganischen oder organischen Säure, z.B. von Borsäure, Ameisensäure, Essigsäure, Propionsäure, n-Butansäure, Benzoesäure, Nicotinsäure, Kohlensäure, Carbaminsäure, Oxalsäure, Bernsteinsäure, Zitronensäure, Rhodanwasserstoffsäure, Phosphorsäure.

Die Umsetzung wird in einem Überschuss des betreffenden Amins der allgemeinen Formel IV oder in einem polaren protischen oder aprotischen Lösungsmittel, z.B. in Methanol, Ethanol, 2-Propanol, Tetrahydrofuran, Dioxan, Dimethylformamid, Dimethylacetamid, Dimethylsulfoxid, Hexamethylphosphorsäuretriamid, Pyridin, Ameisensäure, Essigsäure, Propionsäure, Isobuttersäure, bei Temperaturen zwischen −20 und 160°C, bevorzugt zwischen +110 und 130°C, und gegebenenfalls bei erhöhtem Druck im Autoklaven durchgeführt. Die Verbindungen der allgemeinen Formeln IV, IVa bzw. V werden in der Regel in einem Überschuss von 3 bis 10 Mol — bezogen auf das umzusetzende Substrat der allgemeinen Formeln II bzw. III — verwendet; es können aber auch äquimolare Mengen der Reaktanden eingesetzt werden.

2. Zur Herstellung von Verbindungen der allgemeinen Formel I, in der $R_1$ und $R_2$ Wasserstoffatome bedeuten:

2.1 Durch Umsetzung von 2-Acyl-benzo[b]thiophen-3-olen der allgemeinen Formel II

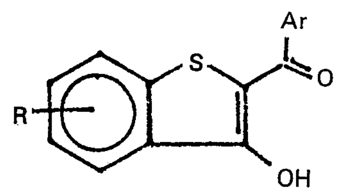

(II)

oder von Enolethern der allgemeinen Formel

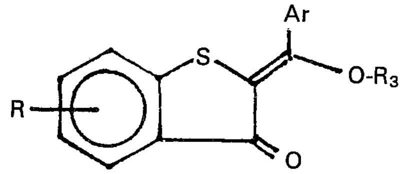

(III)

mit Formamid, wobei in den allgemeinen Formeln II und III Ar, R und $R_3$ wie oben definiert sind.

2.2 Durch Umsetzung von 2-Acyl-3-chlor-benzo-[b]thiophenen der allgemeinen Formel VI

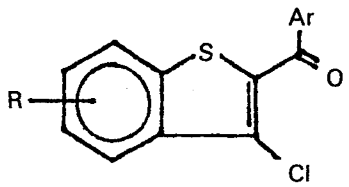

(VI)

mit Ammoniumsalzen der allgemeinen Formel IVb,

$NH_4^{\oplus}$ $B^{\ominus}$ (IVb)

in denen Ar, R und $B^-$ wie oben definiert sind.

Die Umsetzung wird in polaren, protischen oder aprotischen Lösungsmitteln, z.B. in Methanol, Ethanol, 2-Propanol, Tetrahydrofuran, Dioxan, Dimethylformamid, Dimethylacetamid, Pyridin, Dimethylsulfoxid, Hexamethylphosphorsäuretriamid, bevorzugt jedoch in niederen aliphatischen Carbonsäuren, wie Ameisensäure, Essigsäure, Propionsäure, n-Butansäure, bei Temperaturen zwischen 20 und 160°C, bevorzugt 110 bis 130°C, und gegebenenfalls bei erhöhtem Druck im Autoklaven durchgeführt. Ein Überschuss an Formamid bzw. an Ammoniumsalz der allgemeinen Formel IVb bis zu 10 Mol ist für die Ausbeute an einer Verbindung der allgemeinen Formel I vorteilhaft, es können aber auch äquimolare Mengen der Reaktanden VI und IVb bzw. des Formamids eingesetzt werden.

3. Durch Umsetzung von 2-Acyl-benzo[b]thiophen-3-olen der allgemeinen Formel II mit Phosphor-(V)-chlorid, nachfolgende Aminolyse der anfallenden instabilen salzartigen Verbindungen der vermuteten, aber unbewiesenen Formel VII

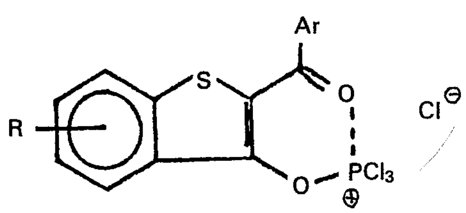

(VII)

mit Aminen der allgemeinen Formel IV, wobei neben den Endprodukten der allgemeinen Formel I nichtstöchiometrisch zusammengesetzte phosphorhaltige Substanzen der denkbaren, aber ebenso unbewiesenen Formel VIII

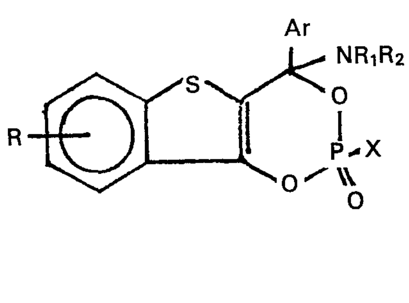

(VIII)

anfallen, deren Hydrolyse mit verdünnten wässrigen Mineralsäuren gleichfalls zu den gewünschten vinylogen Carboxamiden der allgemeinen Formel I in Form der entsprechenden mineralsauren Salze führt.

In den hypothetischen allgemeinen Formeln VII und VIII haben Ar, R, $R_1$ und $R_2$ die eingangs angegebenen Bedeutungen, X bedeutet Chlor und/oder Hydroxy und/oder den Rest $-NR_1R_2$.

Die Umsetzung der 2-Acyl-benzo[b]thiophen-3-ole der allgemeinen Formel II mit Phosphor(V)-chlorid erfolgt in inerten, kohlenwasserstoffähnlichen Lösungsmitteln, wie Petrolether, Benzin, Benzol, Toluol, Xylol, Chlorbenzol, 1,2-Dichlorbenzol und bei Temperaturen zwischen 0 und 40°C, bevorzugt zwischen 15 und 25°C. In Anbetracht ihrer Instabilität erweist sich die baldige weitere Umsetzung der Salze der vermuteten Struktur VII mit Aminen der allgemeinen Formel IV als zweckmässig. Die Amine der Formel IV werden bevorzugt in Form wässriger Lösungen verwendet, können jedoch auch als solche eingesetzt werden. Pro Mol des salzartigen Komplexes der allgemeinen Formel VII verwendet man einen Überschuss von 2 bis 20 Mol des Amins der allgemeinen Formel IV; bei Zusatz von Hilfsbasen wie Natriumcarbonat, Kaliumcarbonat, Natriumhydrogencarbonat, Kaliumhydrogencarbonat oder tert. Aminen, wie Triethylamin, Trimethylamin, 1,5-Diazabicyclo-[4,3,0]non-5-en, 1,8-Diazabicyclo[5,4,0]undec-7-en genügen jedoch auch äquimolare Mengen des Amins der allgemeinen Formel IV. Die Aminolyse, in der Regel als Zweiphasenreaktion durchgeführt, erfolgt bei Temperaturen zwischen 0 und 100°C, bevorzugt 40 und 60°C.

Die stickstoff- und phosphorhaltigen Verbindungen der angenommenen Formel VIII lassen sich durch Behandlung mit verdünnten, wässrigen Mineralsäuren in die entsprechenden Salze der gesuchten vinylogen Carboxamide der allgemeinen Formel I überführen. Als wässriger Mineralsäuren kommen beispielsweise 1 bis 20%ige wässriger Salzsäure, Bromwasserstoffsäure oder Jodwasserstoffsäure, 1- bis 50%ige wässriger Schwefelsäure oder Phosphorsäure in Betracht. Für die Hydrolysereaktion eignen sich Temperaturen zwischen 30 und 100°C, bevorzugt 50 und 70°C. Die anfallenden Salze lassen sich in üblicher Weise durch Behandeln mit Basen, z.B. wässrigen Lösungen von Natriumhydroxid, Kaliumhydroxid, Calciumhydroxyid, Kaliumcarbonat, Natriumcarbonat, Natriumhydrogencarbonat, Ammoniak oder Aminen, wie Methylamin, Triethylamin, in die gewünschten Endprodukte der allgemeinen Formel I überführen.

4. Zur Herstellung von Verbindungen der allgemeinen Formel I, in der $R_1$ und $R_2$ Wasserstoffatome bedeuten:

Durch Reduktion von Verbindungen der allgemeinen Formel IX,

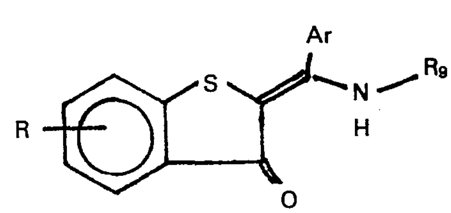

(IX)

in der Ar und R wie oben definiert sind und $R_9$ die Hydroxy-Gruppe oder den Rest $-NR_5R_6$ bedeutet, wobei $R_5$ und $R_6$ entweder gleiche oder voneinander verschiedene Alkylgruppen mit 1 bis 6 Kohlenstoffatomen oder aber $R_5$ ein Wasserstoffatom und $R_6$ ein gegebenenfalls durch Halogenatome oder Alkyl- oder Alkoxygruppen mit 1 bis 3 Kohlenstoffatomen substituierter Phenylrest sind, und R und Ar die eingangs definierten Bedeutungen besitzen.

Als Reduktionsmethoden eignen sich:

4.1. Die Hydrierung in Gegenwart von katalytisch wirkenden Metallen der 8. Nebengruppe des Periodensystems der Elemente: Besonders geeignet sind Palladium- und Platinkatalysatoren, beispielsweise Palladium auf Tierkohle oder feinverteiltes Platin, das in situ aus Platin(IV)-oxid entsteht. Die Hydrierung erfolgt vorzugsweise in polaren Lösungsmitteln wie Eisessig, Propionsäure, Methanol, Ethanol, Dioxan, Tetrahydrofuran und gegebenenfalls in Gegenwart starker Mineralsäuren, z.B. von Perchlorsäure, Schwefelsäure oder Orthophosphorsäure, bei Temperaturen zwischen 0 und 50°C, vorzugsweise jedoch bei Raumtemperatur und bei einem Wasserstoffdruck von 0,5 bis 5 bar; es kann jedoch auch höherer Wasserstoffdruck angewendet werden. Bedeutet in einer Verbindung der allgemeinen Formel IX Ar einen Nitrophenyl-Rest, so wird die Nitrogruppe zur Aminogruppe mitreduziert.

4.2 Die Reduktion mittels nascierendem Wasserstoff und/oder Zinn(II)-chlorid. Hierbei haben sich insbesondere die Reduktion mittels Eisenpulver in Gegenwart von verdünnten oder halbkonzentrierten Mineralsäuren, wie von Salzsäure, Bromwasserstoffsäure, Schwefelsäure, Phosphorsäure, und die Reduktion mittels Zinn(II)-chlorid in Gegenwart von konzentrierter Salzsäure oder Bromwasserstoffsäure bewährt. Bei der Reduktion mittels Eisen werden als Solventien polare, mit Wasser mischbare organische Lösungsmittel wie Methanol, Ethanol, Dioxan, Tetrahydrofuran oder Eisessig bevorzugt und man arbeitet bei Temperaturen zwischen 10 und 100°C, bevorzugt bei Raumtemperatur. Für die Zinn(II)-chlorid-Methode kommen Temperaturen zwischen 0 und 40°C, bevorzugt Raumtemperatur in Betracht und als zusätzliche Lösungsmittel werden die eben genannten verwendet. Bedeutet in einer Verbindung der allgemeinen Formel IX Ar einen Nitrophenyl-Rest, so wird die Nitrogruppe mitreduziert.

4.3. Reduktion mit komplexen Alkalimetallaluminiumhydriden, bevorzugt Lithiumaluminiumhydrid oder Natrium-bis-(2-methoxyethoxy)aluminiumhydrid, unter Verwendung von wasserfreien Ethern, z.B. von Diethylether, Dioxan, Tetrahydrofuran, 1,2-Dimethoxy-ethan, Diethylenglykoldimethylether, Triethylenglykoldimethylether, als Lösungsmittel bei Temperaturen zwischen −20 und +100°C, bevorzugt +20 bis +50°C. Bedeutet in einer Verbindung der allgemeinen Formel IX Ar einen Nitrophenyl-Rest, so wird die Nitro- zur Aminogruppe mitreduziert.

4.4. Reduktion mit Natriumdithionit in wässriger Lösung oder Suspension und in Gegenwart von überschüssigem Alkalihydroxid. Die Reaktion kann mit oder ohne zusätzliche Lösungsmittel, wie z.B. Methanol, Ethanol, Dioxan, Tetrahydrofuran, 1,2-Ethandiol, 1,2-Dimethoxy-ethan oder 2-Ethoxy-ethanol, erfolgen. Die Reaktionstemperatur kann bei 60 bis 120°C liegen, die Siedetemperatur des Wassers wird jedoch bevorzugt. Bedeutet in einer Verbindung der allgemeinen Formel IX Ar einen Nitrophenyl-Rest so wird die Nitro- zur Aminogruppe mitreduziert.

4.5. Die Reduktion mit Eisen(II)-hydroxid in wässriger Lösung oder Suspension und bei Temperaturen zwischen 0 und 100°C, bevorzugt zwischen 15 und 50°C. Die Reaktion kann mit oder ohne zusätzliche Lösungsmittel, wie z.B. Methanol, Ethanol, Dioxan, Tetrahydrofuran, 1,2-Ethanol, 1,2-Dimethoxy-ethan oder 2-Ethoxy-ethanol, erfolgen. Das Eisen(II)-hydroxid wird bevorzugt in situ aus geeigneten Eisen(II)-salzen, z.B. aus Eisen(II)-sulfat-heptahydrat, Eisen(II)-chlorid, Eisen(II)-chlorid-tetrahydrat, Eisen(II)-nitrat-hexahydrat durch Zugabe von Basen, bevorzugt von wässriger Ammoniak-Lösung, erzeugt. Bedeutet in einer Verbindung der allgemeinen Formel IX Ar einen Nitrophenyl-Rest, so wird die Nitro- zur Aminogruppe mitreduziert.

5. Zur Herstellung von Verbindungen der allgemeinen Formel I, in der $R_1$ ein Wasserstoffatom und $R_2$ und R wie oben definiert sind:

Durch Umsetzung von vinylogen Carboxamiden der allgemeinen Formel X,

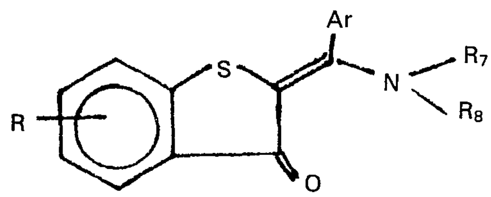

(X)

in der

$R_7$ ein Wasserstoffatom oder die Methylgruppe und

$R_8$ ein Wasserstoffatom, einen geradkettigen oder verzweigten Alkylrest mit bis zu 6 Kohlenstoffatomen, eine 2-Propenyl- oder 2-Propinylgruppe, einen gegebenenfalls methylsubstituierten Cycloalkylrest mit 3 bis 8 Kohlenstoffatomen oder den Rest $-A-R_4$ bedeuten und

Ar, R A und $R_4$ wie eingangs definiert sind, mit Aminen der allgemeinen Formel IV oder mit Ammoniumsalzen der allgemeinen Formel IVa oder mit Harnstoffen der allgemeinen Formel V,

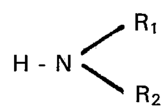

(IV)

$H_2\overset{\oplus}{N}R_1R_2 \quad \overset{\ominus}{B}$

(IVa)

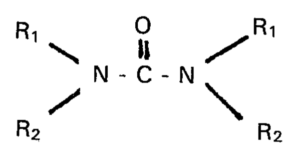

(V)

in denen $R_1$ ein Wasserstoffatom ist und $R_2$ wie oben definiert ist, und B(−) das Anion einer ein- oder

mehrbasischen schwachen bis mittelstarken anorganischen oder organischen Säure, z.B. von Borsäure, Ameisensäure, Essigsäure, Propionsäure, n-Butansäure, Benzoesäure, Nicotinsäure, Kohlensäure, Carbaminsäure, Oxalsäure, Bernsteinsäure, Zitronensäure, Rhodanwasserstoffsäure, Phosphorsäure bedeutet.

Die Umsetzung wird in einem Überschuss des betreffenden Amins der allgemeinen Formel IV oder in einem polaren protischen oder aprotischen Lösungsmittel, z.B. in Methanol, Ethanol, 2-Propanol, Tetrahydrofuran, Dioxan, Dimethylformamid, Dimethylacetamid, Dimethylsulfoxid, Hexamethylphosphorsäuretriamid, Pyridin, Ameisensäure, Essigsäure, Propionsäure, Isobuttersäure, bei Temperaturen zwischen −20 und 160°C, bevorzugt zwischen + 110 und 130°C, und gegebenenfalls bei erhöhtem Druck im Autoklaven durchgeführt. Die Verbindungen der allgemeinen Formeln IV, IVa bzw. V werden in der Regel in einem Überschuss von 3 bis 10 Mol — bezogen auf das umzusetzende Substrat der allgemeinen Formel X — verwendet; es können aber auch äquimolare Mengen der Reaktanden eingesetzt werden.

Die als Ausgangsstoffe verwendeten Verbindungen der allgemeinen Formel II sind teilweise literaturbekannt oder in Analogie zu literaturbekannten Verfahren [K. Görlitzer, Arch. Pharm. *307,* 523 (1974)] herstellbar. Beispielsweise wurden aus den entsprechenden, gegebenenfalls im Benzolkern substituierten Thiosalicylsäuremethylestern und geeigneten 2-Chlor- bzw. 2-Brom-1-aryl-ethanonen die nachfolgenden 2-Acyl-benzo[b]thiophen-3-ole erhalten:

2-(4-Chlorbenzoyl)-benzo[b]thiophen-3-ol, Fp. 124 bis 125°C, (Petrolether/Essigsäureethylester 1:1)
2-(4-Methylbenzoyl)-benzo[b]thiophen-3-ol, Fp. 100-101°C, (Petrolether/Essigsäureethylester 1:1)
2-(3,4-Dichlorbenzoyl)-benzo[b]thiophen-3-ol, Fp. 176-178°C, (Essigsäureethylester)
2-(4-Fluorbenzoyl)-benzo[b]thiophen-3-ol, Fp. 118 bis 120°C, (Essigsäureethylester)
(3-Hydroxy-benzo[b]thien-2-yl)-pyridinyl-methanon, Fp. 139-140°C, (Methanol)
2-Benzoyl-5-chlorbenzo[b]thiophen-3-ol, Fp. 129 bis 132°C, (Methanol)
2-Benzoyl-6-chlorbenzo[b]thiophen-3-ol, Fp. 160 bis 162°C, (Methanol/Essigsäureethylester 1:1)
2-(2-Fluorbenzoyl)-benzo[b]thiophen-3-ol, Fp. 99 bis 100°C, (Petrolether/Essigsäureethylester 1:1)
2-(2-Chlorbenzoyl)-benzo[b]thiophen-3-ol, Fp. 86 bis 88°C, (Methanol)
(3-Hydroxy-benzo[b]thien-2-yl)-2-pyridinyl-methanon, Fp. 167-168°C, (Essigsäureethylester)
2-(2-Chlorbenzoyl)-5-chlorbenzo[b]thiophen-3-ol, Fp. 136-138°C, (Essigsäureethylester/Methanol 1:1)
2-(2-Methylbenzoyl)-benzo[b]thiophen-3-ol, Fp. 126-127°C, (Essigsäureethylester/Methanol 1:1)
2-(3-Methylbenzoyl)-benzo[b]thiophen-3-ol, Fp. 89 bis 92°C, (Petrolether/Essigsäureethylester 1:1)
2-(2-Ethylbenzoyl)-benzo[b]thiophen-3-ol, Fp. 39 bis 40°C, (Methanol)
2-(2-Nitrobenzoyl)-benzo[b]thiophen-3-ol, Fp. 151 bis 153°C, (Essigsäureethylester)
2-(2-Brombenzoyl)-benzo[b]thiophen-3-ol, Fp. 108 bis 110°C, (Methanol)
2-(2-Trifluormethyl-benzoyl)-benzo[b]thiophen-3-ol, Fp. 91-92°C, (Methanol)
2-Benzoyl-5-methylbenzo[b]thiophen-3-ol, Fp. 109 bis 111°C, (Methanol/Essigsäureethylester 1:1)
2-(2-Aminobenzoyl)-benzo[b]thiophen-3-ol, Fp. 129-131°C, (Essigsäureethylester)
2-Benzoyl-6-methylbenzo[b]thiophen-3-ol, Fp. 100 bis 102°C, (Methanol/Essigsäureethylester 1:1)
2-Benzoyl-6-methoxybenzo[b]thiophen-3-ol, Fp. 138-139°C, (Methanol)
2-Benzoyl-4-methylbenzo[b]thiophen-3-ol, Fp. 122 bis 123°C, (Methanol/Essigsäureethylester 1:1).

Die Ausgangsstoffe der allgemeinen Formel III, die neu sind, erhält man nach dem vorstehend beschriebenen Verfahren 3, wenn man anstelle von Aminen der allgemeinen Formel IV ein Gemisch eines Alkohols der allgemeinen Formel $R_3$-OH mit einem tertiären Amin, bevorzugt Triethylamin, verwendet. So wurde beispielsweise hergestellt:

(E)-2-[(Methoxy)phenylmethylen]-benzo[b]thiophen-3(2H)-on, Fp. 136-138°C, (Diisopropylether/Petrolether 1:1)

$C_{16}H_{12}O_2S$ (268,33)

Ber.: C 71,62 H 4,51 S 11,95
Gef.: C 71,46 H 4,83 S 11,98
IR($CH_2Cl_2$): 2845 ($OCH_3$), C = O 1670/cm
UV (Ethanol):
$\lambda_{max}$ (neutral) 255 (E = 0,74), 299 (E = 0,68), 414 (E = 0,25) nm
$\lambda_{max}$ (alkalisch) 257 (E = 0,69), 300 (E = 0,42), 413 (E = 0,15) nm
(Konzentration: 50 µg/ml, Schichtdicke: 0,2 cm)
1H-NMR ($CDCl_3$; 80 MHz): $\delta$ 8,2-7,0 (9H-m, ar.H), 3,69 (3H-s, -O-$CH_3$)
MS: m/e 268 (s), 254, 239, 237, 197, 176 (s), 165, 126.5, 121, 105, 77 (s), 51, 28 (s).

Die Ausgangsstoffe der allgemeinen Formeln IV und V sind literaturbekannt und in der Regel käuflich erhältlich, die der allgemeinen Formeln IVa und IVb durch Reaktion entsprechender Amine der allgemeinen Formel IV mit geeigneten Säuren leicht herstellbar.

Die Ausgangsstoffe der allgemeinen Formel VI sind literaturbekannt [T. Higa und A. J. Krubsack, J. org. Chemistry 41, 3399 (1976)] oder entstehen aus den instabilen Salzen der allgemeinen Formel VII nach längerem Aufbewahren bei Raumtemperatur oder gelindem Erwärmen auf Temperaturen zwischen 30 und 80°C. So erhält man beispielsweise: 2-Benzoyl-3-chlorbenzo[b]thiophen, Fp. 71-73°C, (Cyclohexan).

Die Ausgangsstoffe der allgemeinen Formel IX sind teilweise bekannt [S. B. Arwad und N. F. Abdul-Malik, Austr. J. Chem. *28,* 601-5 (1975)] oder können aus den Verbindungen der allgemeinen Formel II nach dem obigen Verfahren 3 hergestellt werden, indem man die Amine der allgemeinen Formel IV durch Verbindungen der allgemeinen Formel $H_2NR_4$ ersetzt, oder sind — sofern $R_4$ die Bedeutung einer Hydroxy-Gruppe annimmt — aus Verbindungen der allgemeinen Formel II durch Kochen mit Hydroxylamin-hydrochlorid in einem Gemisch von Pyridin und

Ethanol leicht erhältlich. So wurden beispielsweise hergestellt:

(E)-2-[(2-Phenylhydrazino)phenylmethylen]-benzo-[b]thiophen-3(2H)-on, Fp. 168°C, (Methanol/Aceton 1:1)

(E)-2-[(Hydroxyamino)phenylmethylen]-benzo[b]-thiophen-3(2H)-on, Fp. 128-130°C, (Petrolether/Essigsäureethylester 1:1)

$C_{15}H_{11}NO_2S$ (269,32)

| | | | | |
|---|---|---|---|---|
| Ber.: | C 66,90 | H 4,12 | N 5,20 | S 11,90 |
| Gef.: | C 66,90 | H 4,06 | N 5,14 | S 12,06 |

IR ($CH_2Cl_2$): OH 3550; assoziiertes N-H, O-H 3400 bis 2500; C = O 1595; C = C 1610, 1570/cm

UV (Ethanol):

$\lambda_{max}$ (neutral) 257 (E = 0,70), 304-316 (E = 0,34), 330 (E = 0,32) nm

$\lambda_{max}$ (alkalisch) 220 (E = 0,86), 275 (E = 0,47), 405 (E = 0,33) nm

(Konzentration: 50 µg/ml, Schichtdicke: 0,2 cm).

In analoger Weise lassen sich auch die Ausgangsstoffe der allgemeinen Formel X herstellen.

Wie bereits eingangs erwähnt, weisen die neuen Verbindungen der allgemeinen Formel I wertvolle pharmakologische Eigenschaften auf, sie wirken insbesondere auf das Zentralnervensystem, und zeigen vor allem eine antikonvulsive Wirkung.

Beispielsweise wurden die Verbindungen

A = (E)-2[(Amino)phenylmethylen]-benzo[b]thiophen-3(2H)-on

B = (E)-2[(Methylamino)phenylmethylen]-benzo-[b]thiophen-3(2H)-on

C = (E)-2[(Amino)-(2-chlorphenyl)methylen]benzo[b]thiophen-3(2H)-on

D = (E)-2-[[(2-Hydroxyethyl)amino]phenylmethylen]-benzo[b]thiophen-3(2H)-on

E = (E)-2-[(Amino)-(2-methylphenyl)methylen]-benzo[b]thiophen-3(2H)-on

F = (E)-2-[(Ethylamino)phenylmethylen]-benzo-[b]thiophen-3(2H)-on

auf ihre antikonvulsive Wirkung untersucht:

1. *Antikonvulsive Wirkung bei Mäusen*

Die antikonvulsive Wirkung wurde als Hemmung des tonischen Extensorkrampfes der Hinterbeine männlicher Mäuse bei maximalem Elektroschock geprüft.

*Methode:*

Versuchstiere waren männliche SPF-Mäuse (Chbb: NMRI) im Gewicht von 20-26 g, die bis zu 1 Stunde vor oraler Substanzverabreichung Zugang zu Standardnahrung und Trinkwasser hatten.

Die Versuchsdurchführung erfolgte in Anlehnung an SWINYARD, BROWN and GOODMAN [J. Pharmacol. exp. Ther. *106,* 319, (1952)]. Die Elektroschock-Apparatur war nach den Angaben von WOODBURY and DAVENPORT [Arch. int. Pharmacodyn. *92,* 97, (1952)] hergestellt worden. Die elektrischen Reize wurden über mit Wildleder bezogene und mit 0,9%iger NaCl-Lösung befeuchtete Stahlkugelelektroden auf die Kopfseite der Tiere über die Augen gegeben. Es wurde mit Wechselstrom von 50 Hz und 50 mA bei 0,2 sec Dauer gereizt. Bei allen Kontrolltieren trat dabei ein klonischer und danach ein tonischer Streckkrampf der Extremitäten auf; bei durch Antikonvulsiva geschützten Tieren trat der tonische Streckkrampf nicht auf.

Die zu prüfenden Substanzen wurden in 1%igem Tyloseschleim suspendiert und in einem Volumen von 0,1 ml/10 g Maus peroral an 10 Mäuse/Dosis verabreicht. Die Tiere wurden 30, 150 und 300 min nach Substanzverabreichung geschockt und die $ED_{50}$-Werte als 50% der gegen den tonischen Streckkrampf der Hinterextremitäten geschützten Tiere nach dem Verfahren von LITCHFIELD and WILCOXON [J. Pharmacol. exp. Ther. *96,* 99, (1949)] oder graphisch ermittelt.

*Ergebnis:*

| Substanz | $ED_{50}$ mg/kg p.o. 30 | 150 | 300 Min. |
|---|---|---|---|
| A | 12,8 | 37 | 46 |
| B | 90 | 38 | 49 |
| C | 7,0 | 4,4 | 5,0 |
| D | 33 | 41 | 42,5 |
| E | 22,8 | 13,3 | 15,7 |
| F | 22,5 | 54,5 | 79 |

2. *Akute Toxizität*

Es wurde die orientierende akute Toxizität der Substanzen nach Gabe einer Dosis an jeweils eine Gruppe von 6 oder 10 Mäusen mit Körpergewichten zwischen 20 und 26 g bestimmt (Beobachtungszeit: 7 Tage):

| Substanz | Orientierende akute Toxizität ($DL_{50}$) |
|---|---|
| A | 2 400 mg/kg p.o. |
| B | > 1 000 mg/kg p.o. |
| C | 500 bis 1000 mg/kg p.o. |
| D | > 1 000 mg/kg p.o. |
| E | > 1 000 mg/kg p.o. |
| F | > 1 000 mg/kg p.o. |

Die erfindungsgemäss hergestellten neuen Verbindungen der allgemeinen Formel I eignen sich somit insbesondere zur Behandlung von spastischen Zuständen und der Epilepsie und lassen sich hierzu zur pharmazeutischen Anwendung gegebenenfalls in Kombination mit anderen Wirksubstanzen in die üblichen galenischen Zubereitungen wie Tabletten, Dragees, Pulver, Suppositorien oder Suspensionen einarbeiten. Die Einzeldosis beträgt hierbei am Menschen zweckmässigerweise 50-300 mg, vorzugsweise jedoch 50-200 mg.

Die nachfolgenden Beispiele sollen die Erfindung näher erläutern.

*Beispiel 1*

*(E)-2-[(Amino)phenylmethylen]-benzo[b]thiophen-3(2H)-on*

In einem 1-l-Scheidetrichter werden 50,86 g (0,2 Mol) 2-Benzoyl-benzo[b]thiophen-3-ol in 600 $cm^3$ Toluol gelöst und nach Zugabe von 42,0 g (0,2 Mol) Phosphor(V)-chlorid 5 min bei Zimmertemperatur mechanisch gerührt. Die Suspension des entstandenen rot gefärbten Komplexes wird anschliessend unter gutem Rühren in 150 $cm^3$ (ca. 2 Mol) konzentrierten Ammoniak eingebracht, wobei die Reaktionstemperatur durch äussere Kühlung mit Wasser auf 40 bis 50°C einreguliert wird. Man erwärmt noch 1 Stunde auf 50°C, lässt erkalten, gibt Salzsäure zu bis pH 1 erreicht ist, und kocht so lange unter Rückfluss, bis der gelbe Startfleck dünnschichtchromatographisch (Kieselgel-Fertigplatten $F_{254}$ Merck; 1,2-Dichlorethan/Essigsäureethylester/Eisessig (100 : 30 : 3) nicht mehr nachgewiesen werden kann. Dann kühlt man auf +10°C ab, nutscht nach 1stündigem Stehen bei dieser Temperatur ab, wäscht den Niederschlag mit wenig Toluol, dann zur Entfernung anorganischer Salze mit Wasser gründlich durch.

Aus den vereinigten Filtraten wird die orangefarbene Toluolphase abgetrennt, einmal mit 500 $cm^3$ Wasser gewaschen und eingedampft. Der anfallende, teilweise kristallisierende Rückstand wird zweimal mit je 20 $cm^3$ Methanol aufgekocht, dann jeweils auf +10°C gekühlt. Dabei geht (E)-2-[(Amino)phenylmethylen]-benzo[b]thiophen-3(2H)-on-hydrochlorid in Lösung, während das unumgesetzte Ausgangsmaterial ungelöst bleibt.

Die erhaltenen Methanol-Lösungen werden mit der Lösung des oben erhaltenen kristallinen Niederschlags in 25 $cm^3$ Methanol vereinigt, mit 25 $cm^3$ Ammoniak versetzt, wonach das gesuchte Produkt in Form gelber Kristalle ausfällt.

Man nutscht ab, trocknet den Niederschlag im Vakuumtrockenschrank, löst das Produkt in 70 $cm^3$ Essigsäureethylester, gibt so viel Petrolether zu, dass sich gerade noch kein Niederschlag bildet (ca. 40 $cm^3$), setzt 4 g Aktivkohle zu, kocht auf und filtriert heiss. Aus dem erkalteten Filtrat erhält man das reine Endprodukt durch Zugabe von 100 $cm^3$ Petrolether und Kühlen auf +10°C. Nach 2stündigem Stehen nutscht man ab und wäscht den Niederschlag mit ca. 40 $cm^3$ Petrolether gründlich durch. Durch Einengen der vereinigten Mutterlaugen auf ca. 50 $cm^3$ Gesamtvolumen kann weiteres Produkt der gleichen Qualität erhalten werden. Nach dem Trocknen im Vakuum schmelzen die gelben Kristalle bei 121,6-122,5°C.

Ausbeute: 35,6 g (70% der Theorie).

DC-Nachweis:

Stationäre Phase: Kieselgel-Fertigplatten $F_{254}$ Merck

Mobile Phase:

a) 1,2-Dichlorethan: $R_F$ Ausgangsmaterial 0,7
$R_F$ Produkt 0,15

b) 1,2-Dichlorethan/Essigsäureethylester/Eisessig (100 : 30 : 3):
$R_F$ Ausgangsmaterial 0,9
$R_F$ Produkt 0,4

$C_{15}H_{11}NOS$ (253,32)

| | | | | |
|---|---|---|---|---|
| Ber.: | C 71,12 | H 4,38 | N 5,53 | S 12,66 |
| Gef.: | C 71,20 | H 4,59 | N 5,59 | S 12,52 |

IR (KBr): N-H 3475, C = O 1600/cm

UV (Ethanol):

$\lambda_{max}$ 184, 317, 430 nm, Schulter bei 270 nm

$^1$H-NMR ($CDCl_3$): $\delta$ 11,0 (1H, breit, innere H-Brücke), 8,01 (1H-dd, J = 7,6 und 2Hz, ar.H), 7,9-7,1 (8H-m, ar.H), 5,78 (1H-s, austauschbarer H)

$^{13}$C-NMR ($CDCl_3$; 22,63 MHz): $\delta$ 103,66, 185,55 (C = O), 131,28, 123,48, 131,80, 124,20, 144,80, 136,87, 160,07, 134,53, 127,97, 129,40, 125,82.

Bei der auf gleiche Weise wie oben erhaltenen $^{15}$N markierten Verbindung spaltet das Signal für den dem $^{15}$N benachbarten Kohlenstoff auf:
$\delta$ 160,59 (d, $J^{15}$N-C 14,71 Hz).

*Beispiel 2*

*(E)-2-[(Methylamino)phenylmethylen]-benzo[b]-thiophen-3(2H)-on*

Hergestellt analog Beispiel 1 aus 2-Benzoyl-benzo-[b]thiophen-3-ol, Phosphor(V)-chlorid und 40%iger wässriger Methylamin-Lösung in einer Ausbeute von 43% der Theorie.

Fp. 155-156°C (Methanol).

$C_{16}H_{13}NOS$ (267,34)

| | | | | |
|---|---|---|---|---|
| Ber.: | C 71,88 | H 4,90 | N 5,24 | S 11,90 |
| Gef.: | C 72,10 | H 4,75 | N 5,21 | S 12,20 |

*Beispiel 3*

*(E)-2-[(Amino)-(4-chlorphenyl)methylen]-benzo-[b]thiophen-3(2H)-on*

Hergestellt analog Beispiel 1 aus 2-(4-Chlorbenzoyl)-benzo[b]thiophen-3-ol, Phosphor(V)-chlorid und konzentriertem wässrigem Ammoniak in einer Ausbeute von 55% der Theorie.

Fp. 182-183°C (Benzol).

$C_{15}H_{10}ClNOS$ (287,77)

| | | | | | |
|---|---|---|---|---|---|
| Ber.: | C 62,61 | H 3,50 | Cl 12,32 | N 4,87 | S 11,14 |
| Gef.: | C 62,90 | H 3,62 | Cl 12,55 | N 4,76 | S 11,18 |

*Beispiel 4*

*(E)-2-[(Dimethylamino)phenylmethylen]-benzo[b]-thiophen-3(2H)-on*

Hergestellt analog Beispiel 1 aus 2-Benzoyl-benzo[b]thiophen-3-ol, Phosphor(V)-chlorid und 40%-iger wässriger Dimethylamin-Lösung in einer Ausbeute von 7% der Theorie.

Fp. 178-180°C (Essigsäureethylester/Petrolether 1:1).

$C_{17}H_{15}NOS$ (281,38)

| | | | | |
|---|---|---|---|---|
| Ber.: | C 72,57 | H 5,37 | N 4,98 | S 11,39 |
| Gef.: | C 72,17 | H 5,33 | N 4,80 | S 11,37 |

IR ($CH_2Cl_2$): C = O 1610/cm

UV (Ethanol):

$\lambda_{max}$ 270 bis 290 (E = 0,43), 332 (E = 0,42) und 454 (E = 0,37) nm

(Schichtdicke 0,2 cm, Konzentration 50 $\mu$g/ml)

$^1$H-NMR ($CDCl_3$, 80 MHz): $\delta$ 7,99 (1H-dd, J = 7 und 2Hz, ar.H), 7,7-7,2 (8H-m, ar.H), 3,28 (6H-s).

*Beispiel 5*

*(E)-2-[(Amino-(4-methylphenyl)methylen]-benzo-[b]thiophen-3(2H)-on*

Hergestellt analog Beispiel 1 aus 2-(4-Methylbenzoyl)-benzo[b]thiophen-3-ol, Phosphor(V)-chlorid und konzentriertem Ammoniak in einer Ausbeute von 62% der Theorie.

Fp. 147°C (Essigsäureethylester/Petrolether 1:1).

$C_{16}H_{13}NOS$ (267,34)

| | | | | |
|---|---|---|---|---|
| Ber.: | C 71,88 | H 4,90 | N 5,24 | S 11,99 |
| Gef.: | C 71,38 | H 4,82 | N 5,14 | S 12,58 |

*Beispiel 6*

*(E)-2-[(Amino)-(3,4-dichlorphenyl)methylen]-benzo[b]thiophen-3(2H)-on*

Hergestellt analog Beispiel 1 aus 2-(3,4-Dichlorbenzoyl)-benzo[b]thiophen-3-ol, Phosphor(V)-chlorid und konzentriertem Ammoniak in einer Ausbeute von 50% der Theorie.
Fp. 169-170°C (Essigsäureethylester).
$C_{15}H_9Cl_2NOS$ (322,21)
Ber.: C 55,92 H 2,28 Cl 22,01 N 4,35 S 9,95
Gef.: C 55,81 H 3,06 Cl 22,45 N 4,47 S 10,10

*Beispiel 7*

*(E)-2-[(Amino)-(4-fluorphenyl)methylen]-benzo[b]thiophen-3(2H)-on*

Hergestellt analog Beispiel 1 aus 2-(4-Fluorbenzoyl)-benzo[b]thiophen-3-ol, Phosphor(V)-chlorid und konzentriertem Ammoniak in einer Ausbeute von 63% der Theorie. Gelbe, filzige Kristalle vom Fp. 158°C (Methanol).
$C_{15}H_{10}FNOS$ (271,31)
Ber.: C 66,41 H 3,72 N 5,16 S 11,82
Gef.: C 66,51 H 4,00 N 5,23 S 11,92
IR ($CH_2Cl_2$): N-H 3465, C = O 1610/cm
UV (Ethanol):
$\lambda_{max}$ 282, 315, 428 nm, Schulter bei 260 nm
$^1$H-NMR ($CDCl_3$): $\delta$ 11,07 (1H, breit, innere H-Brücke), 8,08 (1H-dd, J = 7,5 und 2Hz, ar.H), 7,95-7,1 (7H-m, ar.H), 5,86 (1H, breit, austauschbarer H).

*Beispiel 8*

*(E)-2-[(Amino)-(4-pyridinyl)methylen]-benzo[b]thiophen-3(2H)-on*

Hergestellt analog Beispiel 1 aus (3-Hydroxybenzo[b]thien-2-yl)-4-pyridinyl-methanon,Phosphor(V)-chlorid und 25%iger wässriger Ammoniak-Lösung in einer Ausbeute von 6% der Theorie.
Fp. 198-200°C (Methanol).
$C_{14}H_{10}N_2OS$ (254,30)
Ber.: C 66,12 H 3,96 N 11,02 S 12,61
Gef.: C 65,99 H 3,92 N 10,80 S 12,30
IR (KBr): N-H breit 3470 bis 2800, C = O 1620/cm
UV (Ethanol):
$\lambda_{max}$ 275-281 (E = 0,50), 310 (E = 0,45), 431 (E = 0,43) nm;
nach Alkalizusatz:
$\lambda_{max}$ 285 (E = 0,80), 395 (E = 0,33) nm
(Konzentration: 50 µg/ml, Schichtdicke: 0,2 cm)
$^1$H-NMR ($CDCl_3/CD_3OD$, 80 MHz): $\delta$ 8,86 (2H-dd, J = 5Hz und 2Hz, $\alpha$ -Pyridyl-H), 8,07 (1H-dd, J = 7 und 2Hz, ar.H), 7,85-7,2 (5H-m, ar.H), 2 austauschbare Wasserstoffatome.

*Beispiel 9*

*(E)-2-[(Amino)phenylmethylen]-5-chlorbenzo[b]thiophen-3(2H)-on*

Hergestellt analog Beispiel 1 aus 2-Benzoyl-5-chlorbenzo[b]thiophen-3-ol, Phosphor(V)-chlorid und konzentriertem Ammoniak in einer Ausbeute von 57% der Theorie.
Fp. 172-173°C (Essigsäureethylester).
$C_{15}H_{10}ClNOS$ (287,77)
Ber.: C 62,61 H 3,50 Cl 12,32 N 4,87 S 11,14
Gef.: C 62,39 H 3,47 Cl 12,31 N 4,77 S 11,45
IR (KBr): N-H (bzw. O-H) 3450, 3330, 3140, C = O 1625/cm
UV (Ethanol):
$\lambda_{max}$ 258 (E = 0,42), 287 (E = 0,53), 322 (E = 0,48), $\lambda$ 4,34 (E = 0,49) nm;
nach Alkalizusatz:
$\lambda_{max}$ 286 (E = 0,62), 320 Schulter (E = 0,23), 393 (E = 0,30), 434 Schulter.
(Konzentration: 50 µg/ml, Schichtdicke: 0,2 cm).

*Beispiel 10*

*(E)-2-[(Amino)phenylmethylen]-6-chlorbenzo[b]thiophen-3(2H)-on*

Hergestellt analog Beispiel 1 aus 2-Benzoyl-6-chlorbenzo[b]thiophen-3-ol, Phosphor(V)-chlorid und konzentriertem Ammoniak in einer Ausbeute von 27% der Theorie.
Fp. 191-192°C (Essigsäureethylester).
$C_{15}H_{10}ClNOS$ (287,77)
Ber.: C 62,61 H 3,50 Cl 12,32 N 4,87 S 11,14
Gef.: C 62,80 H 3,50 Cl 12,40 N 5,11 S 11,08
IR (KBr): N-H 3470 und breite Banden bei 3220 und 3120, C = O 1600/cm
UV (Ethanol):
$\lambda_{max}$ (neutral) 286 (E = 0,64), 309 (E = 0,54), 425 (E = 0,55) nm
$\lambda_{max}$ (alkalisch) 284 (E = 0,67), 308 Schulter (E = 0,33), 405 (E = 0,33), 428 (Schulter) nm
(Konzentration: 50 µg/ml, Schichtdicke: 0,2 cm).

*Beispiel 11*

*(E)-2-[(Amino)-(2-fluorphenyl)methylen]-benzo[b]thiophen-3-(2H)-on*

Hergestellt analog Beispiel 1 aus 2-(2-Fluorbenzoyl)-benzo[b]thiophen-3-ol, Phosphor(V)-chlorid und konzentriertem Ammoniak, jedoch unter Verwendung eines Gemisches von Toluol und Petrolether im Volumenverhältnis 2:1 anstelle von reinem Toluol, in einer Ausbeute von 50% der Theorie. Gelbe Kristalle vom Fp. 156-158°C (Essigsäureethylester/Petrolether 1:1).
$C_{15}H_{10}FNOS$ (271,31)
Ber.: C 66,41 H 3,72 N 5,15 S 11,82
Gef.: C 66,11 H 3,61 N 5,25 S 12,57
IR (KBr): N-H 3475, daneben N-H assoziiert, C = O 1600/cm
UV (Ethanol):
$\lambda_{max}$ (neutral) 258 (Schulter), 281, 312, 424 nm,
$\lambda_{max}$ (alkalisch) 281, 310 (Schulter), 388, 422 (Schulter) nm.

*Beispiel 12*

*(E)-2-[(Amino)-(2-chlorphenyl)methylen]-benzo[b]thiophen-3(2H)-on*

Hergestellt analog Beispiel 1 aus 2-(2-Chlorbenzoyl)-benzo[b]thiophen-3-ol, Phosphor(V)-chlorid und konzentriertem Ammoniak in einer Ausbeute von 33% der Theorie.

Fp. 167-168°C (Essigsäureethylester).
$C_{15}H_{10}ClNOS$ (287,77)
Ber.: C 62,61 H 3,50 Cl 12,32 N 4,87 S 11,14
Gef.: C 63,10 H 3,70 Cl 12,95 N 4,84 S 11,78
IR (KBr): N-H 3470 (daneben assoziiertes N-H), C O = 1600/cm
UV (Ethanol):
$\lambda_{max}$ (neutral) 260 Schulter (E = 0,41), 283 (E = 0,51), 310 (E = 0,49), 422 (E = 0,46) nm
$\lambda_{max}$ (alkalisch) 283 (E = 0,60), 308 Schulter (E = 0,11), 380-385 (E = 0,32) nm
(Konzentration: 50 µg/ml, Schichtdicke: 0,2 cm).

*Beispiel 13*

*(E)-2-[(Amino)-(2-pyridinyl)methylen]-benzo[b]-thiophen-3(2H)-on*

Hergestellt analog Beispiel 1 aus (3-Hydroxybenzo[b]thien-2-yl)-2-pyridinyl-methanon, Phosphor-(V)-chlorid und konzentriertem Ammoniak in einer Ausbeute von 27% der Theorie.
Fp. 128-130°C (Essigsäureethylester).
$C_{14}H_{10}N_2OS$ (254,30)
Ber.: C 66,12 H 3,96 N 11,02 S 12,61
Gef.: C 66,34 H 4,09 N 11,45 S 12,70
IR (KBr): N-H bzw. assoziiertes N-H 3290, C = O ca. 1600/cm
UV (Ethanol):
$\lambda_{max}$ (neutral) 250 Schulter (E = 0,39), 274 (E = 0,58), 329 (E = 0,49) und < 390 nm
$\lambda_{max}$ (alkalisch) 286 (E = 0,65), 325 Schulter (E = 0,30), < 390 nm
(Konzentration: 50 µg/l, Schichtdicke: 0,2 cm).

*Beispiel 14*

*(E)-2-[(Amino)-(2-chlorphenyl)methylen]-5-chlor-benzo[b]thiophen-3(2H)-on*

Hergestellt analog Beispiel 1 aus 2-(2-Chlorbenzoyl)-5-chlorbenzo[b]thiophen-3-ol, Phosphor(V)-chlorid und konzentriertem wässrigem Ammoniak in einer Ausbeute von 40% der Theorie.
Fp. 199-201°C (Essigsäureethylester).
$C_{15}H_9Cl_2NOS$ (322,21)
Ber.: C 55,92 H 2,82 Cl 22,01 N 4,35 S 9,95
Gef.: C 55,67 H 3,09 Cl 21,70 N 4,22 S 10,30
IR ($CH_2Cl_2$): N-H 3475 (daneben N-H assoziiert), C = O 1610/cm
UV (Ethanol):
$\lambda_{max}$ (neutral) 260 (E = 0,34), 293 (E = 0,52), 314 (E = 0,44), 432 (E = 0,44) nm
$\lambda_{max}$ (alkalisch) 293 (E = 0,64), 320 Schulter (E = 0,16), 390 (E = 0,32) nm
(Konzentration: 50 µg/ml, Schichtdicke: 0,2 cm).

*Beispiel 15*

*(E)-2-[[(2-Propenyl)amino]phenylmethylen]-benzo-[b]thiophen-3(2H)-on*

Hergestellt analog Beispiel 1 aus 2-Benzoyl-benzo[b]thiophen-3-ol, Phosphor(V)-chlorid und einer Lösung von 1 Volumenteil Allylamin in 2 Volumenteilen Wasser in einer Ausbeute von 43% der Theorie.
Fp. 104-106°C (Essigsäureethylester).
$C_{18}H_{15}NOS$ (293,39)
Ber.: C 73,69 H 5,15 N 4,78 S 11,18
Gef.: C 73,48 H 5,22 N 4,78 S 11,18
IR ($CH_2Cl_2$): C = C 1645, C = O ca. 1600/cm
UV (Ethanol):
$\lambda_{max}$ (neutral) 265 Schulter (E = 0,42), 286 (E = 0,52), 320 (E = 0,56), 431 (E = 0,60);
auf Alkalizusatz keine Änderung
(Konzentration: 50 µg/ml, Schichtdicke: 0,2 cm)
$^1$H-NMR ($CDCl_3$): $\delta$ 12,0 (1H, breit, innere H-Brükke), 8,05 (1H-dd, J = 7 und 2Hz, ar.H), 7,7-7,15 (8H-m, ar.H), 6,15-5,6 (1H-m, olefinischer H), 5,45-5,05 (2H-m, olefin. H), 4,0-3,7 (2H-m, aliph. $CH_2$).

*Beispiel 16*

*(E)-2-[[[2-(Dimethylamino)ethyl]amino]phenylmethylen]-benzo[b]thiophen-3(2H)-on*

Hergestellt analog Beispiel 1 aus 2-Benzoyl-benzo[b]thiophen-3-ol, Phosphor(V)-chlorid und einer Lösung von 2 Volumina N,N-Dimethylethylendiamin in 3 Volumina Wasser in einer Ausbeute von 42% der Theorie. Orangegelbe Kristalle vom Fp. 123 bis 125°C (Essigsäureethylester).
$C_{19}H_{20}N_2OS$ (324,44)
Ber.: C 70,34 H 6,21 N 8,63 S 9,88
Gef.: C 70,16 H 6,11 N 8,65 S 10,17
IR ($CH_2Cl_2$): NH assoziiert, C = O 1600/cm
UV (Ethanol):
$\lambda_{max}$ (neutral) 287 (E = 0,44), 322 (E = 0,44), 431 (E = 0,49) nm;
auf Zusatz von Alkali keine Veränderung
(Konzentration: 50 µg/ml, Schichtdicke: 0,2 cm).

*Beispiel 17*

*(E)-2-[[[3-(Dimethylamino)propyl]amino]phenylmethylen]-benzo[b]thiophen-3(2H)-on*

Hergestellt analog Beispiel 1 aus 2-Benzoyl-benzo[b]thiophen-3-ol, Phosphor(V)-chlorid und einer wässrigen Lösung von 3-(Dimethylamino)propanamin (hergestellt aus 51 g des Diamins und 70 $cm^3$ Wasser) in einer Ausbeute von 26% der Theorie.
Fp. 90-91°C (Essigsäureethylester/Petrolether 1:1).
$C_{20}H_{22}N_2OS$ (338,47)
Ber.: C 70,97 H 6,55 N 8,28 S 9,47
Gef.: C 70,83 H 6,67 N 8,31 S 9,29

*Beispiel 18*

*(E)-2-[[(2-Hydroxyethyl)amino]phenylmethylen]-benzo[b]thiophen-3(2H)-on*

Hergestellt analog Beispiel 1 aus 2-Benzoyl-benzo[b]thiophen-3-ol, Phosphor(V)-chlorid und einer 40%igen wässrigen Ethanolamin-Lösung in einer Ausbeute von 13% der Theorie.
Fp. 122-123°C (Essigsäureethylester/Petrolether 1:1).
$C_{17}H_{15}NO_2S$ (297,38)
Ber.: C 68,66 H 5,08 N 4,71 S 10,78
Gef.: C 68,65 H 4,89 N 4,71 S 11,00
IR ($CH_2Cl_2$): O-H 3620, N-H oder O-H (assoziiert), 3340 breit, C = O ca. 1600/cm
UV (Ethanol):

λ $_{max}$ (neutral) 226 (E = 0,58), 2,63 (E = 0,68), 336 (E = 0,86), 4,60 (E = 0,50) nm
λ $_{max}$ (alkalisch) 257-261 (E = 0,60), 336 (E = 0,63), 4,59 (E = 0,36) nm.

*Beispiel 19*

*(E)-2-[(Amino)-(2-methylphenyl)methylen]-benzo-[b]thiophen-3(2H)-on*

Hergestellt analog Beispiel 1 aus 2-(2-Methylbenzoyl)-benzo[b]thiophen-3-ol, Phosphor(V)-chlorid und konzentriertem wässrigem Ammoniak in einer Ausbeute von 50% der Theorie.
Fp. 143-144°C (Essigsäureethylester/Petrolether 1:1).
$C_{16}H_{13}NOS$ 267,34)

| | | | | |
|---|---|---|---|---|
| Ber.: | C 71,88 | H 4,90 | N 5,24 | S 11,99 |
| Gef.: | C 72,23 | H 5,04 | N 5,27 | S 11,70 |

IR ($CH_2Cl_2$): 3480 (N-H), O-H assoziiert oder NH assoziiert, ca. 1600/cm (C = O)
UV (Ethanol):
λ $_{max}$ (neutral) 260 Schulter (E = 0,48), 285 (E = 0,59), 311 (E = 0,57), 4,22 (E = 0,59) nm
λ $_{max}$ (alkalisch) 285 (E = 0,64), 310 (E = 0,45), 421 (E = 0,42), Schulter bei 404 (E = 0,40) nm (Konzentration: 50 µg/ml, Schichtdicke: 0,2 cm).

*Beispiel 20*

*(E)-2-[[(Ethyl)amino]phenylmethylen]-benzo[b]thiophen-3(2H)-on*

Hergestellt analog Beispiel 1 aus 2-Benzoyl-benzo[b]thiophen-3-ol, Phosphor(V)-chlorid und 30%iger wässriger Ethylamin-Lösung in einer Ausbeute von 56% der Theorie.
Fp. 115-116°C (Essigsäureethylester/Petrolether 1:1).
$C_{17}H_{15}NOS$ (281,38)

| | | | | |
|---|---|---|---|---|
| Ber.: | C 72,57 | H 5,37 | N 4,98 | S 11,39 |
| Gef.: | C 72,43 | H 5,53 | N 4,89 | S 11,65 |

*Beispiel 21*

*(E)-2-[[(Isopropyl)amino]phenylmethylen]-benzo[b]-thiophen-3(2H)-on*

Hergestellt analog Beispiel 1 aus 2-Benzoyl-benzo[b]thiophen-3-ol, Phosphor(V)-chlorid und 38,5%iger wässriger Isopropylamin-Lösung in einer Ausbeute von 54% der Theorie.
Fp. 112-113°C (Essigsäureethylester/Petrolether 1:1).
$C_{18}H_{17}NOS$ (295,40)

| | | | | |
|---|---|---|---|---|
| Ber.: | C 73,19 | H 5,80 | N 4,74 | S 10,85 |
| Gef.: | C 73,15 | H 5,99 | N 4,61 | S 10,80 |

IR ($CH_2Cl_2$): C = O ca. 1590/cm
UV (Ethanol):
λ $_{max}$ (neutral) 286, 321, 429, Schulter bei 260 nm; auf Zusatz von Alkali keine Verschiebung
$^1$H-NMR ($CDCl_3$, 80 MHz): δ 11,88 (1H, breit, innere H-Brücke), 7,95 (1H-dd, J = 7Hz und 2Hz, ar.H), 7,7-7,15 (8H-m, ar.H), 3,9-3,4 (1H-m, >CH), 1,24 (6H-d, J = 6Hz, $[CH_3]_2$).

*Beispiel 22*

*(E)-2-[(Amino)-(3-methylphenyl)methylen]-benzo-[b]thiophen-3(2H)-on*

Hergestellt analog Beispiel 1 aus 2-(3-Methylbenzoyl)-benzo[b]thiophen-3-ol, Phosphor(V)-chlorid und konzentriertem Ammoniak in einer Ausbeute von 36% der Theorie.
Fp. 114-115°C (Essigsäureethylester/Petrolether 1:1).
$C_{16}H_{13}NOS$ (267,34)

| | | | | |
|---|---|---|---|---|
| Ber.: | C 71,88 | H 4,90 | N 5,24 | S 11,99 |
| Gef.: | C 71,95 | H 4,91 | N 5,56 | S 12,17 |

*Beispiel 23*

*(E)-2-[(Amino)-(2-ethylphenyl)methylen]-benzo[b]-thiophen-3(2H)-on*

Hergestellt analog Beispiel 1 aus 2-(2-Ethylbenzoyl)-benzo[b]thiophen-3-ol, Phosphor(V)-chlorid und konzentriertem Ammoniak in einer Ausbeute von 46% der Theorie.
Fp. 132-133°C (Essigsäureethylester/Petrolether 1:1).
$C_{17}H_{15}NOS$ (281,38)

| | | | | |
|---|---|---|---|---|
| Ber.: | C 72,57 | H 5,37 | N 4,98 | S 11,39 |
| Gef.: | C 72,95 | H 5,58 | N 5,05 | S 11,56 |

IR (KBr): 3500 - 3000 N-H assoziiert, ca. 1600/cm C = O
UV (Ethanol):
λ $_{max}$ (neutral) 260-274 (E = 0,42), 284 (E = 0,52), 312 (E = 0,51), 422 (E = 0,53), Schulter bei 410 nm
λ $_{max}$ (alkalisch) 284 (E = 0,56), 312 (E = 0,41), 422 (E = 0,41), Schultern bei 260 (E = 0,37) und 404 (E = 0,38) nm
(Konzentration: 50 µg/ml, Schichtdicke: 0,2 cm).

*Beispiel 24*

*(E)-2-[[(Methyl)amino]-(2-ethylphenyl)methylen]-benzo[b]thiophen-3(2H)-on*

Hergestellt analog Beispiel 1 aus 2-(2-Ethylbenzoyl)-benzo[b]thiophen-3-ol, Phosphor(V)-chlorid und 25%iger wässriger Methylamin-Lösung in einer Ausbeute von 42% der Theorie.
Fp. 126-127°C (Essigsäureethylester/Petrolether 1:1).
$C_{18}H_{17}NOS$ (295,40)

| | | | | |
|---|---|---|---|---|
| Ber.: | C 73,19 | H 5,80 | N 4,74 | S 10,85 |
| Gef.: | C 73,30 | H 5,67 | N 4,85 | S 10,89 |

IR ($CH_2Cl_2$): C = O ca. 1600/cm
UV (Ethanol):
λ $_{max}$ (neutral) 266-274, 289, 318, 429, Schulter bei 412 nm;
auf Alkalizusatz keine Veränderung
$^1$H-NMR ($CDCl_3$, 80 MHz): δ 11,73 (1H, breit, innere H-Brücke), 7,97 (1H-d, verbreitert, J = 6,5Hz, ar.H), 7,7-7,0 (7H-m, ar.H), 2,83 (3H-d, J = 2,5Hz, N-$CH_3$), 2,64 (2H-q, J = 7,2Hz, -$CH_2$-), 1,19 (3H-t, J = 7,2Hz, C-$CH_3$).

*Beispiel 25*

*(E)-2-[[(Ethyl)amino]-(2-ethylphenyl)methylen]--benzo[b]thiophen-3(2H)-on*

Hergestellt analog Beispiel 1 aus 2-(2-Ethylbenzoyl)-benzo[b]thiophen-3-ol, Phosphor(V)-chlorid

und 25%iger wässriger Ethylamin-Lösung in einer Ausbeute von 2% der Theorie.
Fp. 76-77°C (Diisopropylether/Petrolether 1:2).
$C_{19}H_{19}NOS$ (309,43)

| | | | | |
|---|---|---|---|---|
| Ber.: | C 73,75 | H 6,19 | N 4,53 | S 10,36 |
| Gef.: | C 74,05 | H 6,16 | N 4,58 | S 10,35 |

*Beispiel 26*

*(E)-2-[(Amino)-(2-nitrophenyl)methylen]-benzo[b]-thiophen-3(2H)-on*

Hergestellt analog Beispiel 1 aus 2-(2-Nitrobenzoyl)-benzo[b]thiophen-3-ol, Phosphor(V)-chlorid und konzentriertem wässrigem Ammoniak in einer Ausbeute von 21% der Theorie.
Fp. 185-186°C (Essigsäureethylester).
$C_{15}H_{10}N_2O_3S$ (298,32)

| | | | | |
|---|---|---|---|---|
| Ber.: | C 60,39 | H 3,38 | N 9,39 | S 10,75 |
| Gef.: | C 60,23 | H 3,42 | N 9,34 | S 10,79 |

IR ($CH_2Cl_2$): N-H 3480 (ausserdem N-H assoziiert), C = O ca. 1600, $NO_2$ 1350 und 1515-1535/cm
UV (Ethanol):
$\lambda_{max}$ (neutral) 261 (E = 0,52), 287 (E = 0,51), 313 (E = 0,47), 423 (E = 0,40) nm
$\lambda_{max}$ (alkalisch) 246 (E = 0,35), 286 (E = 0,64), 312 (E = 0,20), 383 (E = 0,33) nm
(Konzentration: 50 µg/ml, Schichtdicke: 0,2 cm).

*Beispiel 27*

*(E)-2-[[(1,1-Dimethylethyl)amino]phenylmethylen]-benzo[b]thiophen-3(2H)-on*

Hergestellt analog Beispiel 1 aus 2-Benzoyl-benzo[b]thiophen-3-ol, Phosphor(V)-chlorid und einer 40%igen wässrigen Lösung von 1,1-Dimethylamin in einer Ausbeute von 57% der Theorie.
Fp. 131-132°C (Essigsäureethylester/Petrolether 1:1).
$C_{19}H_{19}NOS$ (309,43)

| | | | | |
|---|---|---|---|---|
| Ber.: | C 73,75 | H 6,19 | N 4,53 | S 10,36 |
| Gef.: | C 73,40 | H 6,25 | N 4,48 | S 10,34 |

IR ($CH_2Cl_2$): N-H assoziiert, C = O ca. 1600, C = C 1610/cm
UV (Ethanol):
$\lambda_{max}$ (neutral) 264, 288, 324, 416 (Schulter), 432 nm;
auf Alkalizusatz keine Veränderung
$^1H$-NMR ($CDCl_3$, 80 MHz): $\delta$ 12,35 (1H-s, breit, innere H-Brücke), 7,96 (1H-dd, J = 7Hz und 2Hz, ar.H), 7,65-7,1 (8H-m, ar.H), 1,28 (9H-s, $-C[CH_3]_3$).

*Beispiel 28*

*(E)-2-[(Amino)-(2-bromphenyl)methylen]-benzo[b]-thiophen-3(2H)-on*

Hergestellt analog Beispiel 1 aus 2-(2-Brombenzoyl)-benzo[b]thiophen-3-ol, Phosphor(V)-chlorid und konzentriertem wässrigem Ammoniak in einer Ausbeute von 42% der Theorie.
Fp. 143-144°C (Petrolether/Essigsäureethylester 1:1).
$C_{15}H_{10}BrNOS$ (332,22)

| | | | | | |
|---|---|---|---|---|---|
| Ber.: | C 54,23 | H 3,03 | Br 24,05 | N 4,22 | S 9,65 |
| Gef.: | C 54,10 | H 3,04 | Br 24,25 | N 4,13 | S 9,56 |

*Beispiel 29*

*(E)-2-[[(2-Proponyl)amino]phenylmethylen]-benzo-[b]thiophen-3(2H)-on*

Hergestellt analog Beispiel 1 aus 2-Benzoyl-benzo-[b]thiophen-3-ol, Phosphor(V)-chlorid und einer wässrigen Propargylamin-Lösung, die durch Umsetzung einer Lösung von 10 g Propargylamin-hydrochlorid in 10 $cm^3$ Wasser mit der Lösung von 10,6 g wasserfreiem Natriumcarbonat in 20 $cm^3$ Wasser erhalten worden war, in einer Ausbeute von 46% der Theorie.
Fp. 126-128°C (Essigsäureethylester(Petrolether 1:1).
$C_{18}H_{13}NOS$ (291,37)

| | | | | |
|---|---|---|---|---|
| Ber.: | C 74,20 | H 4,50 | N 4,81 | S 11,00 |
| Gef.: | C 74,04 | H 4,47 | N 4,70 | S 11,05 |

IR ($CH_2Cl_2$): -C = C-H 3300, C = O ca. 1600/cm, ausserdem assoziiertes H
UV (Ethanol):
$\lambda_{max}$ (neutral) 266-276 (E = 0,45), 286 (E = 0,48), 319 (E = 0,56), 434 (E = 0,54)
$\lambda_{max}$ (alkalisch) 255 (E = 0,70), 313 (E = 0,34), 434 (E = 0,22)
(Konzentration: 50 ug/ml, Schichtdicke: 0,2 cm)
$^1H$-NMR ($CDCl_3$, 80 MHz): $\delta$ 11,75 (1H, breit, innere H-Brücke), 7,95 (1H-dd, J = 7Hz und 2Hz, ar.H), 7,7-7,1 (8H-m, ar.H), 3,94 (2H-dd, J = 6Hz und 2,5 Hz, $-CH_2-$), 2,32 (1H-t, J = 2,5Hz).

*Beispiel 30*

*(E)-2-[(Amino)phenylmethylen]-benzo[b]thiophen-3(2H)-on*

Die Mischung von 10,0 g (0,0393 Mol) 2-Benzoyl-benzo[b]thiophen-3-ol, 60 $cm^3$ konzentriertem wässrigem Ammoniak und 250 $cm^3$ Ethanol wird nach Aufpressen von 5 bar Ammoniak-Gas 20 Stunden auf eine Temperatur von 120°C erhitzt. Nach dem Erkalten rührt man den Ansatz in 1,5 l Wasser, nutscht das ausgefallene hellgelbe Reaktionsprodukt ab, wäscht es mehrfach mit je 50 $cm^3$ Wasser gründlich durch und trocknet es an der Luft. Man erhält 4,23 g (42% der Theorie) an gelben Kristallen vom Fp. 121-122°C (nach zweimaligem Umkristallisieren aus Petrolether/Essigsäureethylester 1:1 unter Verwendung von Aktivkohle), die nach Elementaranalyse, IR- und UV-Spektrum mit dem nach Beispiel 1 erhaltenen Produkt völlig identisch sind.

Hält man die obige Reaktionsmischung 15 Stunden bei 150°C, so beträgt die Ausbeute 5% der Theorie; hält man 15 Stunden bei 80°C, so sinkt die Ausbeute auf 22% der Theorie; nach 15stündigem Erwärmen auf 40°C und nachfolgendem 10stündigem Erhitzen auf 60°C beträgt die Ausbeute 31% der Theorie.

*Beispiel 31*

*(E)-2-[(Amino)phenylmethylen]-benzo[b]thiophen-3(2H)-on*

Man löst 35,1 g (0,138 Mol) 2-Benzoyl-benzo[b]-thiophen-3-ol in 35 $cm^3$ Eisessig, gibt 39,0 (0,506 Mol) Ammoniumacetat zu und kocht $2^1/_2$ Stunden unter Rückfluss. Die noch warme Mischung wird

in 300 $cm^3$ Eiswasser eingerührt, wobei ein rötlich gefärbtes Produkt ausfällt. Der Feststoff wird abfiltriert, an der Luft getrocknet und einmal aus 60 $cm^3$ Toluol umkristallisiert. Zur weiteren Reinigung löst man in 30 $cm^3$ Essigsäureethylester, gibt so viel Petrolether zu, dass sich gerade noch kein Niederschlag bildet (ca. 20 $cm^3$), setzt 20 g Aktivkohle zu, kocht auf und filtriert heiss. Aus dem erkalteten Filtrat erhält man das reine Endprodukt durch Kühlen auf +10°C und Zugabe von 60 $cm^3$ Petrolether. Nach 2stündigem Stehen nutscht man ab. Durch Einengen der Mutterlaugen auf ein Volumen von 30 $cm^3$ und längeres Kühlen auf +10°C lassen sich weitere 2,0 g des gewünschten Produkts in gleicher Qualität erhalten. Die vereinigten Kristallisate werden mit insgesamt 20 $cm^3$ Petrolether gründlich durchgewaschen und im Umlufttrockenschrank bei +70°C vom Lösungsmittel befreit. Man erhält 23,0 g (66% der Theorie) an dottergelben Kristallen vom Fp. 121,6-122,5°C, die nach Elementaranalyse, IR- und UV-Spektrum mit dem nach Beispiel 1 erhaltenen Prparat identisch sind.

*Beispiel 32*

*(E)-2-[[(Cyclohexyl)amino]phenylmethylen]-benzo-[b]thiophen-3(2H)-on*

Hergestellt analog Beispiel 31 in einer Ausbeute von 56% der Theorie aus 2-Benzoyl-benzo[b]thiophen-3-ol und Cyclohexylamin. Zitronengelbe Kristalle vom Fp. 122-123°C (Petrolether/Essigsäureethylester 85:15).
$C_{21}H_{21}NOS$ (335,46)
Ber.: C 75,19 H 6,31 N 4,18 S 9,56
Gef.: C 74,93 H 6,27 N 4,17 S 9,54
IR ($CH_2Cl_2$): C = O ca. 1600/cm, C = C 1610/cm
UV (Ethanol):
$\lambda_{max}$ (neutral) 288 (E = 0,47), 323 (E = 0,46), 432 (E = 0,50);
auf Alkalizusatz keine Verschiebung
$^1$H-NMR ($CDCl_3$, 80 MHz): $\delta$ 12,3-11,8 (H-m, breit, innere H-Brücke), 7,98 (1H-dd, J = 7Hz und 2Hz, ar.H), 7,7-7,1 (8H-m, ar.H), 3,6-3,1 (1H-m, $\lessgtr$CH), 2,1-1,0 (10H-m, Cyclohexyl).

*Beispiel 33*

*(E)-2-[(Amino)phenylmethylen]-benzo[b]thiophen-3(2H)-on*

Hergestellt analog Beispiel 31, jedoch unter Verwendung von (E)-2-[(Methoxy)phenylmethylen]-benzo[b]thiophen-3(2H)-on anstelle von 2-Benzoyl-benzo[b]thiophen-3-ol und Verkürzung der Reaktionsdauer auf 10 min, in einer Ausbeute von 72% der Theorie. Dottergelbe Kristalle vom Fp. 121-122°C, nach DC, Mischschmelzpunkt, Elementaranalyse und IR-Spektrum völlig identisch mit einem nach Beispiel 1 erhaltenen Präparat.

*Beispiel 34*

*(E)-2-[(Amino)phenylmethylen]-benzo[b]thiophen-3(2H)-on*

Man kocht die Mischung von 10,0 g (0,0373 Mol) (E)-2-[(Methoxy)phenylmethylen]-benzo[b]thiophen-3(2H)-on, 75 $cm^3$ konzentriertem Ammoniak und 250 $cm^3$ Methanol 10 min unter Rückfluss, rührt den Ansatz nach dem Erkalten in1,5 l Eiswasser, nutscht das ausgefallene Reaktionsprodukt ab, wäscht es gründlich mit Wasser und trocknet es an der Luft. Zur Reinigung löst man das Rohprodukt in 100 $cm^3$ Ethylacetat, gibt so lange etherische Chlorwasserstoff-Lösung zu, bis sich kein Niederschlag mehr bildet, filtriert und wäscht den Filterrückstand mit Ethylacetat aus. Der Feststoff wird in 100 $cm^3$ Wasser suspendiert und ammoniakalisch gestellt, wonach man abermals abnutscht. Nach dem Trocknen und Umkristallisieren aus Petrolether/Essigsäureethylester erhält man 4,7 g (50% der Theorie) der Titelverbindung vom Fp. 121-122°C, nach DC, Mischschmelzpunkt und Elementaranalyse identisch mit einem nach Beispiel 1 erhaltenen Produkt.

*Beispiel 35*

*(E)-2-[(Amino)phenylmethylen]-benzo[b]thiophen-3(2H)-on*

Eine Mischung aus 35,0 g (0,138 Mol) 2-Benzoyl-benzo[b]thiophen-3-ol, 24,0 g (0,311 Mol) Ammoniumacetat und 25 $cm^3$ Dimethylsulfoxid wird 1 Stunde bei einer Reaktionstemperatur von 125°C gerührt. Die erkaltete Reaktionsmischung rührt man in ein Gemisch von 150 $cm^3$ Petrolether und 300 $cm^3$ Eiswasser ein, nutscht die erhaltene Suspension ab und wäscht den Filterrückstand gründlich mit Wasser. Zur Reinigung nimmt man das getrocknete Rohprodukt in 40 $cm^3$ Essigsäureethylester auf, gibt so viel Petrolether zu, dass sich gerade noch kein Niederschlag bildet (ca. 20 $cm^3$), setzt 5 g Aktivkohle zu, kocht auf und filtriert heiss. Aus dem erkalteten Filtrat erhält man das reine Produkt durch Zugabe von 60 $cm^3$ Petrolether und Kühlen auf +10°C. Durch Einengen der Mutterlaugen auf ca. 30 $cm^3$ Gesamtvolumen kann weiteres Produkt der gleichen Qualität erhalten werden. Man wäscht die vereinigten Kristallisate mit Petrolether, trocknet im Vakuum und erhält 25,2 g (72% der Theorie) der gesuchten Verbindung vom Fp. 121,6-122,5°C, nach Dünnschichtchromatogramm, Mischschmelzpunkt und IR-Spektrum identisch mit einem nach Beispiel 1 erhaltenen Präparat.

*Beispiel 36*

*(E)-2-[[[2-(4-Morpholinyl)ethyl]amino]phenylmethylen]-benzo[b]thiophen-3(2H)-on*

Hergestellt analog Beispiel 35 aus 2-Benzoyl-benzo[b]thiophen-3-ol und 2-(4-Morpholinyl)-ethylamin anstelle von Ammoniumacetat in einer Ausbeute von 62% der Theorie.
$C_{21}H_{22}N_2O_2S$ (366,48)
Ber.: C 68,83 H 6,05 N 7,64 S 8,75
Gef.: C 68,60 H 6,20 N 7,70 S 8,80
IR ($CH_2Cl_2$): N-H assoziiert 3200-3600, C = O ca. 1600/cm
UV (Ethanol):
$\lambda_{max}$ (neutral) 275 (Schulter), 288, 322, 433 nm;
auf Alkalizusatz keine Verschiebung
$^1$H-NMR ($CDCl_3$, 80 MHz): 11,84 (1H, breit, innere

H-Brücke), 7,97 (1H-dd, J = 6,5 und 2H, ar.H), 7,7-7,1 (8H-m, ar.H), 3,9-3,6 (4H-m, $-CH_2O-CH_2-$), 3,5-3,2 (2H-m, $-N-CH_2-$), 2,7-2,3 (6H-m,
$-CH_2-N-CH_2-$)
I
$CH_2-$

*Beispiel 37*

*(E)-2-[[(Propyl)amino]phenylmethylen]-benzo[b]-thiophen-3(2H)-on*

Hergestellt analog Beispiel 35 aus 2-Benzoyl-benzo[b]thiophen-3-ol und n-Propylammoniumacetat in einer Ausbeute von 68% der Theorie.
Fp. 124-125°C (Petrolether/Essigsäureethylester 1:1).
$C_{18}H_{17}NOS$ (295,40)

| | | | | |
|---|---|---|---|---|
| Ber.: | C 73,19 | H 5,80 | N 4,74 | S 10,85 |
| Gef.: | C 72,80 | H 5,88 | N 4,89 | S 10,95 |

*Beispiel 38*

*(E)-2-[[[(n-Butyl)amino]phenylmethylen]-benzo[b]-thiophen-3(2H)-on*

Hergestellt analog Beispiel 35 aus 2-Benzoyl-benzo[b]thiophen-3-ol und n-Butylammoniumacetat in einer Ausbeute von 64% der Theorie.
Fp. 106-107°C (Petrolether/Essigsäureethylester 1:1).
$C_{19}H_{19}NOS$ (309,43)

| | | | | |
|---|---|---|---|---|
| Ber.: | C 73,75 | H 6,19 | N 4,53 | S 10,36 |
| Gef.: | C 73,40 | H 6,13 | N 4,64 | S 10,44 |

*Beispiel 39*

*(E)-2-[[(2-Methylpropyl)amino]phenylmethylen]-benzo[b]thiophen-3(2H)-on*

Hergestellt analog Beispiel 35 aus 2-Benzoyl-benzo[b]thiophen-3-ol und (2-Methylpropyl)ammoniumacetat in einer Ausbeute von 69% der Theorie.
Fp. 125-126°C (Essigsäureethylester/Petrolether 1:2).
$C_{19}H_{19}NOS$ (309,43)

| | | | | |
|---|---|---|---|---|
| Ber.: | C 73,75 | H 6,19 | N 4,53 | S 10,36 |
| Gef.: | C 76,60 | H 6,21 | N 4,68 | S 10,30 |

IR ($CH_2Cl_2$): N-H assoziiert 3200-3600, C = O ca. 1600/cm
UV (Ethanol):
$\lambda_{max}$ (neutral) 270 (Schulter), 288, 323, 432 nm; auf Alkalizusatz keine Verschiebung
$^1$H-NMR ($CDCl_3/D_2O$, 80 MHz): 7,94 (1H-dd, J = 6,5Hz und 2Hz, ar.H), 7,7-7,1 (8Hz-m, ar.H), 3,05 (2H-d, J = 6,5 Hz, $N-CH_2-C$), 2,1-1,5 (1H-m, 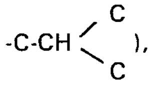 ), 0,95 (6H-d, J = 6,5Hz, $-C[CH_3]_2$).

*Beispiel 40*

*(E)-2-[(Dimethylamino)phenylmethylen]-benzo[b]-thiophen-3(2H)-on*

Hergestellt analog Beispiel 31 aus 2-Benzoyl-benzo[b]thiophen-3-ol und Dimethylammoniumacetat in einer Ausbeute von 18% der Theorie.
Fp. 179-180°C (Essigsäureethylester/Petrolether 1:2).

Das Produkt ist dünnschichtchromatographisch, nach Elementaranalyse und IR-Spektrum mit einem nach Beispiel 4 hergestellten Präparat identisch.

*Beispiel 41*

*(E)-2-[(Amino)phenylmethylen]-benzo[b]thiophen-3(2H)-on*

5,0 g (0,0197 Mol) 2-Benzoyl-benzo[b]thiophen-3-ol, 12,0 g (0,156 Mol) Ammoniumacetat und 5 cm$^3$ 1,2-Ethandiol werden 2 Stunden unter Rühren auf 130°C erhitzt. Man rührt die erkaltete Mischung in 50 cm$^3$ Eiswasser ein, nutscht ab und reinigt den Filterrückstand säulenchromatographisch an Kieselgel unter Verwendung von 1,2-Dichlorethan zum Eluieren. Man erhält nach abschliessendem Umkristallisieren aus Petrolether/Essigsäureethylester (2:1) 2,5 g (50% der Theorie) dottergelbe Kristalle vom Fp. 121-122°C, nach DC, Mischschmelzpunkt und Elementaranalyse identisch mit einem nach Beispiel 1 erhaltenen Präparat.

*Beispiel 42*

*(E)-2-[(Amino)phenylmethylen]-benzo[b]thiophen-3(2H)-on*

Hergestellt analog Beispiel 41 aus 2-Benzoyl-benzo[b]thiophen-3-ol, jedoch unter Verwendung von Harnstoff anstelle von Ammoniumacetat, in einer Ausbeute von 55% der Theorie.
Fp. 121-122°C (Petrolether/Essigsäureethylester 2:1).
Nach DC, Mischschmelzpunkt und IR-Spektrum identisch mit einem nach Beispiel 1 hergestellten Präparat.

*Beispiel 43*

*(E)-2-[(Amino)phenylmethylen]-benzo[b]thiophen-3(2H)-on*

5,0 g (0,0186 Mol) (E)-2-[(Methoxy)phenylmethylen]-benzo[b]thiophen-3(2H)-on und 10,0 g (0,167 Mol) Harnstoff werden gut verrieben und 10 min auf eine Temperatur zwischen 125 und 130°C erhitzt. Das erkaltete Produkt reinigt man säulenchromatographisch wie in Beispiel 41 beschrieben und erhält 3,84 g (81% der Theorie) an dottergelben Kristallen vom Fp. 121-122°C (Petrolether/Essigsäureethylester 2:1). Nach DC, Mischschmelzpunkt, Elementaranalyse und IR-Spektrum identisch mit einem nach Beispiel 1 hergestellten Präparat.

*Beispiel 44*

*(E)-[(Amino)phenylmethylen]-benzo[b]thiophen-3(2H)-on*

Setzt man 2-Benzoyl-benzo[b]thiophen-3-ol mit den nachfolgend angegebenen Reagenzien anstelle von Ammoniumacetat unter den Bedingungen von Beispiel 35 um, so erhält man die Titelverbindung, charakterisiert jeweils durch Dünnschichtchromatogramm, Mischschmelzpunkt, Elementaranalyse und IR-Spektrum, in den rechts angegebenen Ausbeuten:

Ammoniumoxalat 42% der Theorie

| | |
|---|---|
| Ammoniumcarbonat | 64% der Theorie |
| Ammoniumformiat | 74% der Theorie |
| Ammoniumpropionat | 66% der Theorie |
| Ammoniumcarbamat | 59% der Theorie |
| Diammoniumhydrogencitrat | 61% der Theorie |

*Beispiel 45*

*(E)-2-[(Amino)phenylmethylen]-benzo[b]thiophen--3(2H)-on*

Setzt man 2-Benzoyl-benzo[b]thiophen-3-ol mit den nachfolgend genannten Reagenzien anstelle von Ammoniumacetat unter den Bedingungen von Beispiel 31 um, so erhält man die Titelverbindung vom Fp. 121-122°C (Petrolether/Essigsäureethylester 2:1) in den rechts angegebenen Ausbeuten:

| | |
|---|---|
| Ammoniumformiat | 78% der Theorie |
| Formamid | 70% der Theorie |
| Ammoniumthiocyanat | 33% der Theorie |
| Triammoniumphosphat | 27% der Theorie |

*Beispiel 46*

*(E)-2-[(Methylamino)phenylmethylen]-benzo[b]-thiophen-3(2H)-on*

Hergestellt analog Beispiel 31 aus 2-Benzoyl-benzo[b]thiophen-3-ol, jedoch unter Verwendung von N,N'-Dimethylharnstoff anstelle von Ammoniumacetat, in einer Ausbeute von 66% der Theorie.
Fp. 156-157°C (Essigsäureethylester). Nach Dünnschichtchromatogramm, Mischschmelzpunkt, Elementaranalyse und IR-Spektrum identisch mit dem nach Beispiel 2 erhaltenen Präparat.

*Beispiel 47*

*(E)-2-[(Amino)-(2-bromphenyl)methylen]-benzo[b]-thiophen-3(2H)-on*

Hergestellt analog Beispiel 31 aus 2-(2-Brombenzoyl)-benzo[b]thiophen-3-ol und Formamid anstelle von Ammoniumacetat in einer Ausbeute von 10% der Theorie.
Fp. 143-144°C (Petrolether/Essigsäureethylester 1:1). Nach Dünnschichtchromatogramm, Mischschmelzpunkt, Elementaranalyse und IR-Spektrum identisch mit dem nach Beispiel 28 erhaltenen Produkt.

*Beispiel 48*

*(E)-2-[(Amino)phenylmethylen]-benzo[b]thiophen--3(2H)-on*

Hergestellt analog Beispiel 35, jedoch unter Verwendung von 2-Benzoyl-3-chlorbenzo[b]thiophen anstelle von 2-Benzoyl-benzo[b]thiophen-3-ol, in einer Ausbeute von 9% der Theorie.
Fp. 120-121°C (Essigsäureethylester/Petrolether 1:2). Nach Dünnschichtchromatogramm, IR- und UV-Spektrum identisch mit einem nach Beispiel 1 erhaltenen Produkt.

*Beispiel 49*

*(E)-2-[(Amino)phenylmethylen]-benzo[b]thiophen--3(2H)-on*

0,5 g (0,00186 Mol) (E)-2-[(Hydroxyamino)phenylmethylen]-benzo[b]thiophen-3(2H)-on, gelöst in 10 $cm^3$ Eisessig, werden in Gegenwart von 0,4 g Platin(IV)-oxid 2$^1/_2$ Stunden bei einem Wasserstoffdruck von 5 bar katalytisch hydriert. Nach Beendigung der Wasserstoffaufnahme rührt man die vom Katalysator befreite Lösung in 100 $cm^3$ Wasser, extrahiert erschöpfend mit Ether, wäscht die vereinigten Etherauszüge nacheinander mit Wasser, gesättigter wässriger Natriumhydrogencarbonatlösung und abermals mit Wasser, trocknet sie über Natriumsulfat und kristallisiert den nach dem Verjagen des Lösungsmittels verbleibenden kristallisierenden Rückstand unter Verwendung von Aktivkohle zweimal aus einem Gemisch von Petrolether und Essigsäureethylester (2:1) um. Man erhält 0,37 g (79% der Theorie) an dottergelben Kristallen vom Fp. 121-122°C. Nach Dünnschichtchromatogramm, Mischschmelzpunkt und IR-Spektrum identisch mit einem nach Beispiel 1 erhaltenen Präparat.

*Beispiel 50*

*(E)-2-[(Amino)phenylmethylen]-benzo[b]thiophen--3(2H)-on*

Hergestellt analog Beispiel 49 aus (E)-2-[(Phenylhydrazino)phenylmethylen]-benzo[b]thiophen--3(2H)-on in einer Ausbeute von 48% der Theorie.
Fp. 121-122°C (Essigsäureethylester/Petrolether 1:2). Nach Dünnschichtchromatogramm, Mischschmelzpunkt und IR-Spektrum identisch mit einem nach Beispiel 1 erhaltenen Präparat.

*Beispiel 51*

*(E)-2-[(Amino)phenylmethylen]-benzo[b]thiophen--3(2H)-on*

Man suspendiert 0,5 g (0,00145 Mol) (E)-2-[(2--Phenylhydrazino)phenylmethylen]-benzo[b]thiophen-3(2H)-on (Fp. 168°C nach dem Umkristallisieren aus Methanol/Aceton 1:1) in 5 $cm^3$ 2%iger wässriger Natronlauge, fügt 1,0 g (0,00476 Mol) Natriumdithionit-dihydrat zu und kocht 2 Stunden unter Rückfluss. Nach dem Erkalten extrahiert man die Mischung mit 1,2-Dichlorethan, vertreibt das organische Lösungsmittel und reinigt den verbleibenden Rückstand säulenchromatographisch an Kieselgel unter Verwendung von 1,2-Dichlorethan zum Eluieren. Nach abschliessendem Umkristallisieren aus Petrolether/Essigsäureethylester (2:1) erhält man 0,22 g (54% der Theorie) an leuchtend gelben Kristallen vom Fp. 121-122°C, nach Dünnschichtchromatogramm, Mischschmelzpunkt und IR-Spektrum identisch mit dem nach Beispiel 1 synthetisierten Produkt.

*Beispiel 52*

*(E)-2-[(Amino)phenylmethylen]-benzo[b]thiophen--3(2H)-on*

Hergestellt analog Beispiel 51, jedoch unter Verwendung von (E)-2-[(Hydroxyamino)phenylmethylen]-benzo[b]thiophen-3(2H)-on als Ausgangsmaterial, in einer Ausbeute von 47%.
Fp. 121-122°C (Petrolether/Essigsäureethylester 2:1). Nach Dünnschichtchromatogramm, Mischschmelzpunkt und IR-Spektrum identisch mit einem nach Beispiel 1 erhaltenen Präparat.

*Beispiel 53*

*(E)-2-[(Amino)phenylmethylen]-benzo[b]thiophen--3(2H)-on*

Man löst 0,5 g (0,00145 Mol) (E)-2-[(2-Phenylhydrazino)phenylmethylen]-benzo[b]thiophen-3(2H)--on in 10 $cm^3$ Eisessig und fügt 5 $cm^3$ 20%ige Salzsäure und 1,0 g Eisenpulver zu. Durch äussere Kühlung mit Wasser sorgt man für Einhaltung einer Reaktionstemperatur von +15 bis +20°C. Nach 1 Stunde wird der Ansatz wie in Beispiel 49 aufgearbeitet. Man erhält 0,33 g (90% der Theorie) der Titelverbindung vom Fp. 121-122°C (Petrolether/Essigsäureethylester 2:1). Nach Dünnschichtchromatogramm, Mischschmelzpunkt und Elementaranalyse identisch mit einem nach Beispiel 1 erhaltenen Produkt.

*Beispiel 54*

*(E)-2-[(Amino)-(2-trifluormethylphenyl)methylen]--benzo[b]thiophen-3(2H)-on*

Hergestellt analog Beispiel 1 aus 2-(2-Trifluormethyl-benzoyl)-benzo[b]thiophen-3-ol, Phosphor(V)-chlorid und konzentriertem Ammoniak in einer Ausbeute von 6% der Theorie.
Fp. 148-149°C (Petrolether/Essigsäureethylester 2:1).
$C_{16}H_{10}F_3NOS$ (321,31)

| | | | |
|---|---|---|---|
| Ber.: | C 59,81 | H 3,14 | N 4,36 |
| Gef.: | C 59,99 | H 3,49 | N 4,27 |

*Beispiel 55*

*(E)-2-[(Amino)-(2-aminophenyl)methylen]-benzo-[b]thiophen-3(2H)-on*

0,5 g (0,00168 Mol) (E)-2-[(Amino)-(2-nitrophenyl)methylen]-benzo[b]thiophen-3(2H)-on werden in einem Gemisch aus 10 $cm^3$ Essigsäureethylester und 10 $cm^3$ Tetrahydrofuran gelöst und nach Zusatz von 0,5 g Raney-Nickel bei Zimmertemperatur und einem Wasserstoffdruck von 5 bar bis zur Beendigung der Wasserstoffaufnahme hydriert. Man filtriert vom Katalysator ab, dampft das Lösungsmittel bei vermindertem Druck ab und kristallisiert den verbleibenden Rückstand zweimal unter Verwendung von Aktivkohle aus Tetrahydrofuran/Petrolether (1:1) um. Man erhält 330 mg (73% der Theorie) an gelben Kristallen vom Fp. 186-187°C (Petrolether/Tetrahydrofuran 1:1).
$C_{15}H_{12}N_2OS$ (268,34)

| | | | | |
|---|---|---|---|---|
| Ber.: | C 67,14 | H 4,51 | N 10,44 | S 11,95 |
| Gef.: | C 66,73 | H 4,84 | N 9,82 | S 11,75 |

*Beispiel 56*

*(E)-2-[[(Cyclopropyl)amino]phenylmethylen]-benzo-[b]thiophen-3(2H)-on*

Hergestellt analog Beispiel 31 in einer Ausbeute von 25% der Theorie aus 2-Benzoyl-benzo[b]thiophen-3-ol und Cyclopropylamin. Dottergelbe Kristalle vom Fp. 131-132°C (Petrolether/Essigsäureethylester 1:1).
$C_{18}H_{15}NOS$ (293,39)

| | | | | |
|---|---|---|---|---|
| Ber.: | C 73,69 | H 5,15 | N 4,77 | S 10,93 |
| Gef.: | C 73,28 | H 5,38 | N 4,75 | S 11,10 |

IR ($CH_2Cl_2$): Innere H-Brücke, C = O 1595/cm
UVV (Ethanol):
$\lambda_{max}$ 266-276 (E = 0,44), 286 (E = 0,46), 321 (E = 0,49), 435 (E = 0,54) nm
(Konzentration: 50 µg/ml, Schichtdicke: 0,2 cm)
$^1$H-NMR ($CDCl_3/CD_3OD$, 80 MHz): $\delta$ 7,96 (1H-dd, J = 7Hz und 2Hz, ar.H), 7,6-7,1 (8H-m, ar.H), 2,8-2,4 (1H-m, N-CH), 0,8-0,5 (4H-m, 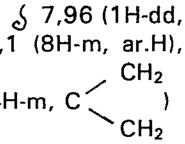 )

*Beispiel 57*

*(E)-2-[(Amino)phenylmethylen]-benzo[b]thiophen--3(2H)-on*

0,5 g (0,00186 Mol) (E)-2-[(Hydroxyamino)phenylmethylen]-benzo[b]thiophen-3(2H)-on werden in 20 $cm^3$ Tetrahydrofuran gelöst, nacheinander mit der Lösung von 1,05 g (0,038 Mol) Eisen(II)-sulfat--heptahydrat in 2 $cm^3$ Wasser und 1 Tropfen konzentrierter Salzsäure und mit 3 $cm^3$ konzentriertem Ammoniak versetzt und 3 Stunden bei Zimmertemperatur gerührt, abschliessend noch 5 min auf 90°C erwärmt. Die erkaltete Mischung wird in 100 $cm^3$ Wasser eingerührt und erschöpfend mit Ether extrahiert. Man wäscht die Etherauszüge mit Wasser, trocknet über Natriumsulfat und kristallisiert den nach dem Vertreiben des Lösungsmittels verbleibenden kristallisierenden Rückstand unter Verwendung von Aktivkohle zweimal aus einem Gemisch von Petrolether und Essigsäureethylester (2:1) um. Man erhält 0,24 g (51% der Theorie) an dottergelben Kristallen vom Fp. 121-122°C. Nach Dünnschichtchromatogramm, Mischschmelzpunkt, Elementaranalyse und IR-Spektrum identisch mit einem nach Beispiel 1 erhaltenen Präparat.

*Beispiel 58*

*(E)-2-[(Amino)phenylmethylen]-benzo[b]thiophen--3(2H)-on*

0,5 g (0,00145 Mol) (E)-2-[(2-Phenylhydrazino)-phenylmethylen]-benzo[b]thiophen-3(2H)-on, 1,0 g (0,00443 Mol) Zinn(II)-chlorid-dihydrat und 5 $cm^3$ konzentrierte Salzsäure werden bei Zimmertemperatur 24 Stunden lang magnetisch gerührt. Die erhaltene klare Lösung wird im Vakuum zu einer viskosen Paste eingedampft, dann mit 10 $cm^3$ Wasser versetzt und durch tropfenweise Zugabe von 10%iger Ammoniak-Lösung auf pH 7 gebracht. Man zieht die erhaltene Suspension erschöpfend mit Essigsäureethylester aus, trocknet die vereinigten organischen Extrakte über Natriumsulfat, engt bei vermindertem Druck ein und reinigt den verbleibenden Rückstand säulenchromatographisch an Kieselgel unter Verwendung von 1,2-Dichlorethan zum Eluieren. Nach abschliessendem Umkristallisieren aus Petrolether/Essigsäureethylester (2:1) erhält man 0,21 g (57% der Theorie) an dottergelben Kristallen vom Fp. 121-122°C. Nach Dünnschichtchromatogramm, Mischschmelzpunkt und Elementaranalyse identisch mit einem nach Beispiel 1 synthetisierten Präparat.

*Beispiel 59*

*(E)-2-[(Amino)phenylmethylen]-benzo[b]thiophen--3(2H)-on*

0,5 g (0,00145 Mol) (E)-2-[(2-Phenylhydrazino)-phenylmethylen]-benzo[b]thiophen-3(2H)-on werden in 10 $cm^3$ wasserfreiem Tetrahydrofuran gelöst und nach Zugabe von 0,1 g (0,00264 Mol) Lithiumtetrahydridoaluminat 30 min bei Raumtemperatur gerührt. Zur Zersetzung des überschüssigen Lithiumtetrahydridoaluminats gibt man nacheinander 10 Tropfen Wasser, 10 Tropfen 15%ige wässrige Natriumhydroxid-Lösung und wiederum 50 Tropfen Wasser zu, kocht auf, filtriert, befreit das Filtrat vom Lösungsmittel und reinigt den Rückstand wie in Beispiel 58 beschrieben. Man erhält 0,085 g (23% der Theorie) an dottergelben Kristallen vom Fp. 120-121°C. Nach Dünnschichtchromatogramm und Mischschmelzpunkt identisch mit einem nach Beispiel 1 erhaltenen Präparat.

*Beispiel 60*

*(E)-2-[(Amino)phenylmethylen]-5-methylbenzo[b]-thiophen-3(2H)-on*

Hergestellt analog Beispiel 35 aus 2-Benzoyl-5--methylbenzo[b]thiophen-3-ol und Ammoniumacetat in einer Ausbeute von 51% der Theorie.
Fp. 153-154°C (Toluol).
$C_{16}H_{13}NOS$ (267,34)

| | | | | |
|---|---|---|---|---|
| Ber.: | C 71,88 | H 4,90 | N 5,24 | S 11,90 |
| Gef.: | C 72,10 | H 5,08 | N 5,22 | S 12,15 |

IR ($CH_2Cl_2$): NH 3470, C = O 1600(cm
UV (Ethanol):
$\lambda_{max}$ (neutral) 266-271 (E = 0,53), 325 (E = 0,58), 435 (E = 0,51) nm
$\lambda_{max}$ (alkalisch) 279 (E = 0,52), 325 (E = 0,47), 433 (E = 0,38) nm
(Konzentration: 50 µg/ml, Schichtdicke: 0,2 cm)
$^1$H-NMR ($CDCl_3$, 80 MHz): $\delta$ 10,94 (1H, breit, innere H-Brücke), 7,9-7,15 (8H-m, ar.H), 5,88 (1H, breit, austauschbarer H), 2,46 (3H-s).

*Beispiel 61*

*(E)-2-[(Amino)phenylmethylen]-6-methylbenzo[b]-thiophen-3(2H)-on*

Hergestellt analog Beispiel 31 aus 2-Benzoyl-6--methylbenzo[b]thiophen-3-ol und Ammoniumacetat in einer Ausbeute von 57% der Theorie.
Fp. 166-167°C (Essigsäureethylester/Petrolether 1:1).
$C_{16}H_{13}NOS$ (267,34)

| | | | | |
|---|---|---|---|---|
| Ber.: | C 71,88 | H 4,90 | N 5,24 | S 11,99 |
| Gef.: | C 71,70 | H 4,94 | N 5,05 | S 11,95 |

IR ($CH_2Cl_2$): NH 3470, C = O 1600/cm
UV (Ethanol):
$\lambda_{max}$ (neutral) 283 (E = 0,57), 321 (E = 0,55), 427 (E= 0,51) nm
(Konzentration: 50 µg/ml, Schichtdicke: 0,2 cm).

*Beispiel 62*

*(E)-2-[(Amino)phenylmethylen]-6-methoxybenzo-[b]thiophen-3(2H)-on*

Hergestellt analog Beispiel 31 aus 2-Benzoyl-6--methoxybenzo[b]thiophen-3-ol und Ammoniumacetat in einer Ausbeute von 64% der Theorie.
Fp. 147-149°C (Toluol/Petrolether 1:1).
$C_{16}H_{13}NO_2S$ (283,34)

| | | | | |
|---|---|---|---|---|
| Ber.: | C 67,82 | H 4,62 | N 4,94 | S 11,32 |
| Gef.: | C 67,77 | H 4,69 | N 5,26 | S 11,85 |

*Beispiel 63*

*(E)-2-[(Amino)phenylmethylen]-4-methylbenzo[b]-thiophen-3(2H)-on*

Hergestellt analog Beispiel 31 aus 2-Benzoyl-4--methylbenzo[b]thiophen-3-ol und Ammoniumacetat in einer Ausbeute von 50% der Theorie.
Fp. 198-200°C (Diisipropylether/Essigsäureethylester 2:1).
$C_{16}H_{13}NOS$ (267,34)

| | | | |
|---|---|---|---|
| Ber.: | C 71,88 | H 4,90 | N 5,24 |
| Gef.: | C 71,75 | H 5,01 | N 5,23 |

IR ($CH_2Cl_2$): NH 3460, C = O 1600/cm
UV (Ethanol):
$\lambda_{max}$ (neutral) 272-275 (E = 0,60), 315 (E = 0,46), 433 (E = 0,59) nm
$\lambda_{max}$ (alkalisch) 272-275 (E = 0,60), 315 (E = 0,44), 433 (E = 0,55) nm
(Konzentration: 50 µg/ml, Schichtdicke: 0,2 cm).

*Beispiel 64*

*(E)-2-[(Methylamino)phenylmethylen]-benzo[b]-thiophen-3(2H)-on*

Man löst 2,53 g (0,01 Mol) (E)-2-[(Amino)phenylmethylen]-benzo[b]thiophen-3(2H)-on in 10 ml Eisessig, gibt 20,0 g (0,22 Mol) Methylammoniumacetat zu und kocht 5 Stunden unter Rückfluss. Die noch warme Mischung wird in 100 ml Eiswasser eingerührt, wobei ein rötlich gefärbtes Produkt ausfällt. Der Feststoff wird abfiltriert, an der Luft getrocknet und zwecks Abtrennung geringer Mengen Ausgangsmaterial unter Verwendung von anfangs 1,2-Dichlorethan, dann von 1,2-Dichlorethan/Aceton (Volumenverhältnis 95:5) als Eluentien säulenchromatographisch an Kieselgel gereinigt. Der nach Eindampfen der Eluate verbleibende dottergelbe Rückstand wird noch zweimal aus Methanol umkristallisiert. Man erhält 2,05 g (77% der Theorie) an gelben Kristallen vom Fp. 155-156°C, die nach Elementaranalyse, IR- und UV-Spektrum und Mischschmelzpunkt mit einem nach Beispiel 2 erhaltenen Präparat identisch sind.

*Beispiel 65*

*(E)-2-[(Methylamino)phenylmethylen]-benzo[b]-thiophen-3(2H)-on*

Die Mischung aus 2,53 g (0,01 Mol) (E)-2-[(Amino)phenylmethylen]-benzo[b]thiophen-3(2H)-on, 40,0 g (0,44 Mol) Methylammoniumacetat und 10 ml Dimethylsulfoxid wird 5 Stunden auf eine Reaktionstemperatur von 130 bis 140°C erwärmt. Man arbeitet wie in Beispiel 64 auf und erhält die gewünschte Verbindung vom Fp. 154-155°C (Methanol) in einer Ausbeute von 58% der Theorie. Das Präparat ist mit einer nach Beispiel 2 erhaltenen Probe nach Elementaranalyse und dünnschichtchromatographischer vergleichender Untersuchung identisch.

*Beispiel 66*

*(E)-2-[(Amino)phenylmethylen]-benzo[b]thiophen--3(2H)-on*

Man löst 2,67 g (0,01 Mol) (E)-2-[(Methylamino)-phenylmethylen]-benzo[b]thiophen-3(2H)-on in 10 ml Eisessig, gibt 25,0 g (0,32 Mol) Ammoniumacetat zu und kocht 15 min unter Rückfluss. Nach Aufarbeitung wie in Beispiel 64 erhält man 1,85 g (73% der Theorie) an gelben Kristallen vom Fp. 121-122°C (Petrolether/Essigsäureethylester 2:1), die nach Mischschmelzpunkt, IR- und UV-Spektrum mit einem nach Beispiel 1 erhaltenen Präparat identisch sind.

*Beispiel 67*

*(E)-2-[(Amino)phenylmethylen]-benzo[b]thiophen--3(2H)-on*

Man verfährt wie in Beispiel 66, verwendet jedoch anstelle von Ammoniumacetat 0,32 Mol Harnstoff. Nach entsprechender Aufarbeitung erhält man 1,92 g (76% der Theorie) an gelben Kristallen vom Fp. 121-122°C (Petrolether/Essigsäureethylester 2:1), die nach Elementaranalyse, Mischschmelzpunkt und dünnschichtchromatographischer Untersuchung mit einem nach Beispiel 1 erhaltenen Präparat übereinstimmt.

Die Verbindungen der allgemeinen Formel I lassen sich in die üblichen pharmazeutischen Zubereitungsformen, wie Tabletten, Dragees, einarbeiten. Die Einzeldosis beträgt für Erwachsene 20 bis 200 mg, vorzugsweise 40 bis 100 mg, die Tagesdosis im allgemeinen 40 bis 400 mg, vorzugsweise 80 bis 200 mg. Im folgenden werden einige Beispiele hierfür genannt:

*Beispiel I*

Tabletten enthaltend 50 mg (E)-2-[(Amino)phenylmethylen]-benzo[b]thiophen-3(2H)-on

Zusammensetzung:

| | |
|---|---|
| Wirkstoff | 50 mg |
| Milchzucker | 148 mg |
| Kartoffelstärke | 60 mg |
| Magnesiumstearat | 2 mg |
| | 260 mg |

Man rührt in eine 10%ige Kartoffelstärkelösung die Wirksubstanz und den Milchzucker und granuliert das Gemenge durch ein Sieb der Maschenweite 1,5 mm, trocknet das Granulat und reibt es erneut durch dasselbe Sieb, man fügt das Magnesiumstearat zu und verpresst das Granulat zu Tabletten. Gewicht der Tablette: 260 mg.

*Beispiel II*

Dragees enthaltend 50 mg (E)-2-[(Methylamino)-phenylmethylen]-benzo[b]thiophen-3(2H)-on

Man stellt analog zu Beispiel I ein Granulat her, welches zu Dragee-Kernen verpresst wird. Die Kerne werden durch eine Hülle versehen, die im wesentlichen aus Zucker und Talk besteht. Die Hülle wird anschliessend mit Bienenwachs poliert.

Gewicht des Kerns: 260 mg

Gewicht des Dragees: 300 mg.

**Patentansprüche für die Vertragsstaaten:**
BE, CH, LI, DE, FR, GB, IT, LU, NL, SE

1. Neue vinyloge Carboxamide der allgemeinen Formel I

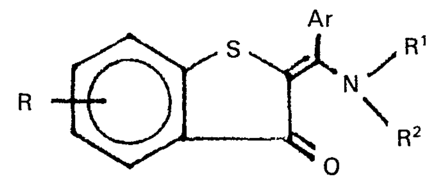

(I)

in der

Ar einen Phenylrest, der durch 1 oder 2 Halogenatome, Alkylgruppen mit 1 bis 3 Kohlenstoffatomen, die Amino-, Nitro- oder Cyangruppe oder einen Trifluormethylrest substituiert sein kann, oder einen Pyridinylrest,

R ein Wasserstoff- oder Chloratom oder die Methylgruppe,

$R_1$ ein Wasserstoffatom oder die Methylgruppe,

$R_2$ ein Wasserstoffatom, einen geradkettigen oder verzweigten Alkylrest mit höchstens 6 Kohlenstoffatomen, einen 2-Propenyl- oder 2-Propinylrest, einen gegebenenfalls methylsubstituierten Cycloalkylrest mit 3 bis höchstens 8 Kohlenstoffatomen oder den Rest -A-$R_4$ bedeuten,

wobei

A eine Alkylengruppe mit 2 oder 3 Kohlenstoffatomen und

$R_4$ die Hydroxygruppe, der Methylamino-, Dimethylamino-, N-Methyl-ethylamino-, Diethylamino-, Pyrrolidino-, Piperidino-, Hexamethylenimino-, Morpholino- oder 4-Methyl-1-piperazinyl-Rest darstellt.

2. Neue vinyloge Carboxamide der allgemeinen Formel I gemäss Anspruch 1, in der

Ar einen Phenylrest, der in o-Stellung durch Fluor, Chlor, Brom, Methyl oder Ethyl substituiert sein kann,

R ein Wasserstoffatom,

$R_1$ ein Wasserstoffatom oder die Methylgruppe,

$R_2$ ein Wasserstoffatom, die Methyl-, Ethyl-, 2-Hydroxyethyl- oder 2-(Dimethylamino)ethylgruppe bedeuten.

3. (E)-2-[(Amino)phenylmethylen]-benzo[b]thiophen-3(2H)-on.

4. (E)-2-[(Methylamino)phenylmethylen]-benzo-[b]thiophen-3(2H)-on.

5. (E)-2-[[(2-Hydroxyethyl)amino]phenylmethylen]-benzo[b]thiophen-3(2H)-on.

6. (E)-2-[(Amino)-(2-methylphenyl)methylen]-wird;

7. (E)-2-[(Ethylamino)phenylmethylen]-benzo-[b]thiophen-3(2H)-on.

8. Arzneimittel, enthaltend eine Verbindung der Ansprüche 1 bis 7 neben einem oder mehreren inerten Trägerstoffen und/oder Verdünnungsmitteln.

9. Arzneimittel gemäss Anspruch 8 zur Behandlung von spastischen Zuständen und der Epilepsie.

10. Verfahren zur Herstellung von neuen vinylogen Carboxamiden gemäss den Ansprüchen 1 bis 7, dadurch gekennzeichnet, dass

a) ein 2-Acyl-benzo[b]thiophen-3-ol der allgemeinen Formel II

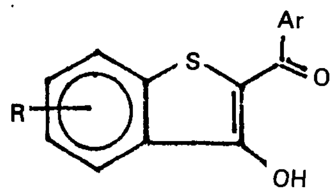

(II)

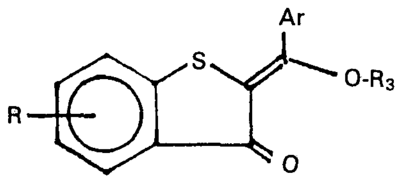

(III)

mit Aminen der allgemeinen Formel IV oder mit Ammoniumsalzen der allgemeinen Formel IVa oder mit Harnstoffen der allgemeinen Formel V

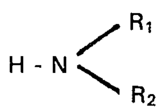

(IV)

$\overset{\oplus}{H_2NR_1R_2}$ $\overset{\ominus}{B}$

(IVa)

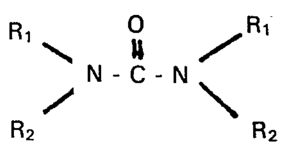

(V)

in welchen Ar, R, $R_1$ und $R_2$ die in Anspruch 1 angegebenen Bedeutungen haben, $R_3$ eine Alkyl-, Alkenyl- oder eine gegebenenfalls im Benzolkern durch Halogenatome, Nitro- oder Methylgruppen substituierte Phenylalkylgruppe mit maximal 20 Kohlenstoffatomen und $B^-$ das Anion einer ein- oder mehrbasischen schwachen bis mittelstarken anorganischen oder organischen Säure bedeuten, in einem Lösungsmittel oder in einem Überschuss des Amins der allgemeinen Formel IV bei Temperaturen zwischen −20 und +160°C umgesetzt wird oder

b) zur Herstellung von Verbindungen der allgemeinen Formel I in der $R_1$ und $R_2$ Wasserstoffatome bedeuten, ein 2-Acyl-benzo[b]thiophen-3-ol der allgemeinen Formel II

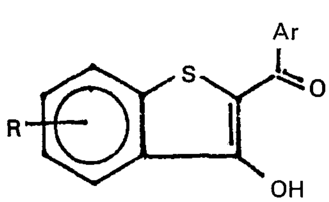

(II)

oder ein Enolether der allgemeinen Formel III

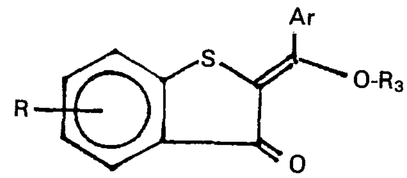

(III)

mit Formamid oder ein 2-Acyl-3-chlor-benzo[b]thiophen der allgemeinen Formel VI

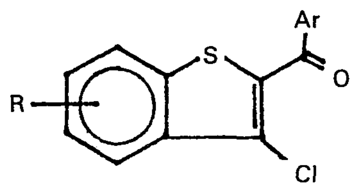

(VI)

mit einem Ammoniumsalz der allgemeinen Formel IVb

$[NH_4]^+$ $B^-$ (IVb)

wobei in diesen Formeln Ar und R wie oben definiert sind und $B^-$ das Anion einer ein- oder mehrbasischen schwachen bis mittelstarken anorganischen oder organischen Säure bedeutet, in einem Lösungsmittel bei Temperaturen zwischen 20 und 160°C umgesetzt werden, oder

c) ein 2-Acyl-benzo[b]thiophen-3-ol der allgemeinen Formel II

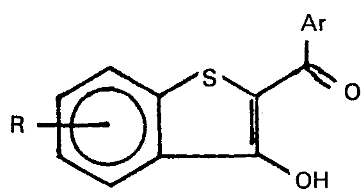

(II)

in der Ar und R wie eingangs erwähnt definiert sind, mit Phosphorpentachlorid in einem inerten Lösungsmittel bei Temperaturen zwischen 0 und 40°C umgesetzt, das dabei entstehende Produkt anschliessend mit einem Amin der allgemeinen Formel IV

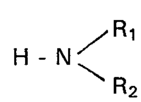

(IV)

in der $R_1$ und $R_2$ die oben angegebenen Bedeutungen besitzen, zwischen 0 und 100°C behandelt und das sich bildende Produkt schliesslich mit wässriger, verdünnter Mineralsäure bei Temperaturen zwischen 30 und 100°C in das Salz des Endprodukts der allgemeinen Formel I übergeführt wird, woraus durch Behandeln mit einer Base die Verbindung der allgemeinen Formel I freigesetzt wird, oder

d) zur Herstellung einer Verbindung der allgemeinen Formel I, in der $R_1$ und $R_2$ Wasserstoffatome bedeuten, eine Verbindung der allgemeinen Formel IX

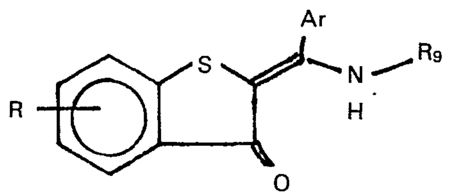

(IX)

in der Ar und R wie oben definiert sind und $R_9$ die Hydroxygruppe oder den Rest $-NR_5R_6$ bedeutet, worin $R_5$ und $R_6$ entweder gleiche oder voneinander verschiedene Alkylgruppen mit 1 bis 6 Kohlenstoffatomen oder aber $R_5$ ein Wasserstoffatom und $R_6$ eine gegebenenfalls durch Halogenatome oder Alkyl- oder Alkoxygruppen mit 1 bis 3 Kohlenstoffatomen substituierte Phenylgruppe bedeuten, durch Hydrierung in Gegenwart von katalytisch wirkenden Metallen der 8. Nebengruppe des Periodensystems in Lösungsmitteln bei Temperaturen zwischen 0 und 50°C, durch Behandeln mit nascierendem Wasserstoff und/oder Zinn(II)-chlorid in polaren Lösungsmitteln in Gegenwart einer Säure bei Temperaturen zwischen 10 und 100°C, durch Behandeln mit komplexen Alkalimetallaluminiumhydriden in wasserfreien Ethern bei Temperaturen zwischen −20 und +100°C, durch Behandeln mit Natriumdithionit in wässriger Lösung oder Suspension und in Gegenwart von Alkalihydroxid bei Temperaturen zwischen 60 und 120°C oder durch Behandeln mit Eisen(II)-hydroxid in wässriger Lösung oder Suspension bei Temperaturen zwischen 0 und 100°C reduziert wird;

e) zur Herstellung einer Verbindung der allgemeinen Formel I, in der $R_1$ ein Wasserstoffatom und $R_2$ und R die in Anspruch 1 angegebene Bedeutung haben, ein Carboxamid der allgemeinen Formel X

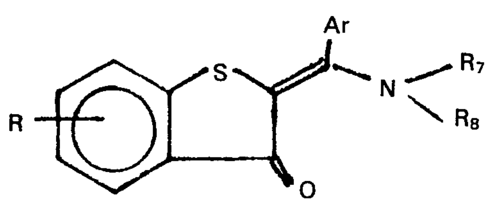

(X)

in der

$R_7$ ein Wasserstoffatom oder die Methylgruppe,

$R_8$ ein Wasserstoffatom, einen geradkettigen oder verzweigten Alkylrest mit bis zu 6 Kohlenstoffatomen, einen 2-Propenyl- oder 2-Propinylrest, einen gegebenenfalls methylsubstituierten Cycloalkylrest mit 3 bis 8 Kohlenstoffatomen oder den Rest $-A-R_4$ bedeuten und

Ar, R, A und $R_4$ wie oben definiert sind mit einem Amin der allgemeinen Formel IV, mit einem Ammoniumsalz der allgemeinen Formel IVa oder mit einem Harnstoff der allgemeinen Formel V

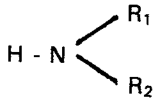

(IV)

$H_2\overset{\oplus}{N}R_1R_2 \quad \overset{\ominus}{B}$

(IVa)

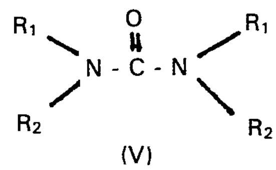

(V)

in denen $R_1$ und $R_2$ wie oben definiert sind und $B^-$ das Anion einer ein- oder mehrbasischen anorganischen oder organischen Säure darstellt, in Gegenwart eines Überschusses des betreffenden Amins der allgemeinen Formel IV oder in einem polaren protischen oder aprotischen Lösungsmittel bei Temperaturen zwischen −20 und 160°C umgesetzt wird.

**Patentanspruch für den Vertragsstaat:** AT

Verfahren zur Herstellung von neuen vinylogen Carboxamiden der allgemeinen Formel I

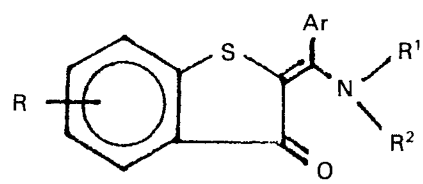

(I)

in der

Ar einen Phenylrest, der durch 1 oder 2 Halogenatome, Alkylgruppen mit bis zu 3 Kohlenstoffatomen, die Amino-, Nitro- oder Cyangruppe oder die Trifluormethylgruppe substituiert sein kann, oder einen Pyridinylrest,

R ein Wasserstoffatom oder Chloratom oder die Methylgruppe,

$R_1$ ein Wasserstoffatom oder die Methylgruppe,

$R_2$ ein Wasserstoffatom, einen geradkettigen oder verzweigten Alkylrest mit höchstens 6 Kohlenstoffatomen, einen 2-Propenyl- oder 2-Propinylrest, einen gegebenenfalls methylsubstituierten Cycloalkylrest mit 3 bis höchstens 8 Kohlenstoffatomen oder den Rest $-A-R_4$ bedeuten,

wobei

A eine Alkylengruppe mit 2 oder 3 Kohlenstoffatomen,

$R_4$ die Hydroxygruppe, der Methylamino-, Dimethylamino-, N-Methyl-ethylamino-, Diethylamino-, Pyrrolidino-, Piperidino-, Hexamethylenimino-, Morpholino- oder 4-Methyl-1-piperazinyl-Rest darstellt, dadurch gekennzeichnet, dass

a) ein 2-Acyl-benzo[b]thiophen-3-ol der allgemeinen Formel II

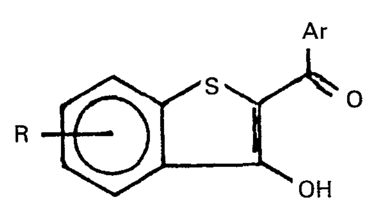

(II)

oder ein Enolether der allgemeinen Formel III

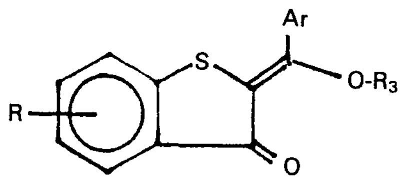

(III)

mit Aminen der allgemeinen Formel IV, mit Ammoniumsalzen der allgemeinen Formel IVa oder mit Harnstoffen der allgemeinen Formel V

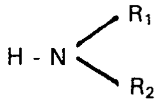

$H_2\overset{\oplus}{N}R_1R_2 \quad \overset{\ominus}{B}$

(IV) (IVa)

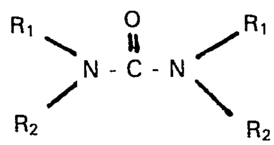

(V)

in welchen Ar, R, $R_1$ und $R_2$ die eingangs angegebenen Bedeutungen haben, $R_3$ eine Alkyl-, Alkenyl- oder eine gegebenenfalls im Benzolkern durch Halogenatome, Nitro- oder Methylgruppen substituierte Phenylalkylgruppe mit maximal 20 Kohlenstoffatomen und $B^-$ das Anion einer ein- oder mehrbasischen schwachen bis mittelstarken anorganischen oder organischen Säure bedeuten, in einem Lösungsmittel oder in einem Überschuss des Amins der allgemeinen Formel IV bei Temperaturen zwischen $-20$ und $+160°C$ umgesetzt wird oder

b) zur Herstellung von Verbindungen der allgemeinen Formel I in der $R_1$ und $R_2$ Wasserstoffatome bedeuten, ein 2-Acyl-benzo[b]thiophen-3-ol der allgemeinen Formel II

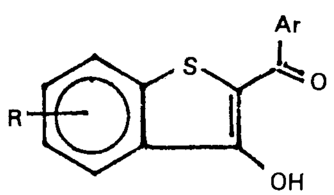

(II)

oder ein Enolether der allgemeinen Formel III

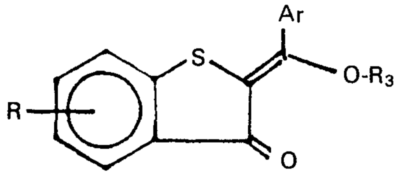

(III)

mit Formamid oder ein 2-Acyl-3-chlor-benzo[b]thiophen der allgemeinen Formel VI

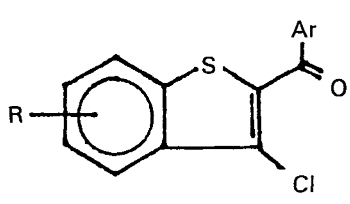

(VI)

mit einem Ammoniumsalz der allgemeinen Formel IVb

$$[NH_4]^+ \quad B^- \qquad (IVb)$$

wobei in diesen Formeln Ar und R wie oben definiert sind und $B^-$ das Anion einer ein- oder mehrbasischen schwachen bis mittelstarken anorganischen oder organischen Säure bedeutet, in einem Lösungsmittel bei Temperaturen zwischen 20 und 160°C umgesetzt werden, oder

c) ein 2-Acyl-benzo[b]thiophen-3-ol der allgemeinen Formel II

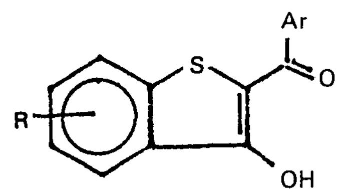

(II)

in der Ar und R wie eingangs erwähnt definiert sind, mit Phosphorpentachlorid in einem inerten Lösungsmittel bei Temperaturen zwischen 0 und 40°C umgesetzt, das dabei entstehende Produkt anschliessend mit einem Amin der allgemeinen Formel IV

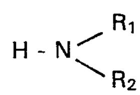

(IV)

in der $R_1$ und $R_2$ die oben angegebenen Bedeutungen besitzen, zwischen 0 und 100°C behandelt und das sich bildende Produkt schliesslich mit wässriger, verdünnter Mineralsäure bei Temperaturen zwischen 30 und 100°C in das Salz des Endprodukts der allgemeinen Formel I übergeführt wird, woraus durch Behandeln mit einer Base die Verbindung der allgemeinen Formel I freigesetzt wird, oder

d) zur Herstellung einer Verbindung der allgemeinen Formel I, in der $R_1$ und $R_2$ Wasserstoffatome bedeuten, eine Verbindung der allgemeinen Formel IX

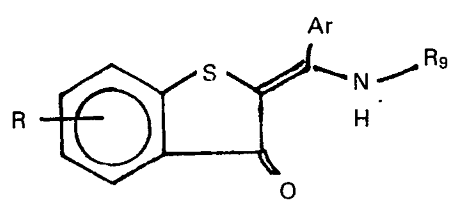

(IX)

in der Ar und R wie oben definiert sind und $R_9$ die Hydroxygruppe oder den Rest $-NR_5R_6$ bedeutet, worin $R_5$ und $R_6$ entweder gleiche oder voneinander verschiedene Alkylgruppen mit 1 bis 6 Kohlenstoffatomen oder aber $R_5$ ein Wasserstoffatom und $R_6$ eine gegebenenfalls durch Halogenatome oder Alkyl- oder Alkoxygruppen mit 1 bis 3 Kohlenstoffatomen substituierte Phenylgruppe bedeuten, durch Hydrierung in Gegenwart von katalytisch wirkenden Metallen der 8. Nebengruppe des Periodensystems in Lösungsmitteln bei Temperaturen zwischen 0 und 50°C, durch Behandeln mit nascierendem Wasserstoff und/oder Zinn(II)-chlorid in polaren Lösungsmit-

teln in Gegenwart einer Säure bei Temperaturen zwischen 10 und 100°C, durch Behandeln mit komplexen Alkalimetallaluminiumhydriden in wasserfreien Ethern bei Temperaturen zwischen −20 und +100°C, durch Behandeln mit Natriumdithionit in wässriger Lösung oder Suspension und in Gegenwart von Alkalihydroxid bei Temperaturen zwischen 60 und 120°C oder durch Behandeln mit Eisen(II)-hydroxid in wässriger Lösung oder Suspension bei Temperaturen zwischen 0 und 100°C reduziert wird;

e) zur Herstellung einer Verbindung der allgemeinen Formel I, in der $R_1$ ein Wasserstoffatom und $R_2$ und R die in Anspruch 1 angegebene Bedeutung besitzt, ein Carboxamid der allgemeinen Formel X

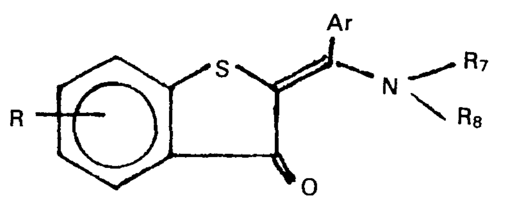

(X)

in der

$R_7$ ein Wasserstoffatom oder die Methylgruppe,

$R_8$ ein Wasserstoffatom, einen geradkettigen oder verzweigten Alkylrest mit bis zu 6 Kohlenstoffatomen, einen 2-Propenyl- oder 2-Propinylrest, einen gegebenenfalls methylsubstituierten Cycloalkylrest mit 3 bis 8 Kohlenstoffatomen oder den Rest $-A-R_4$ bedeuten und

Ar, R, A und $R_4$ wie oben definiert sind mit einem Amin der allgemeinen Formel IV, mit einem Ammoniumsalz der allgemeinen Formel IVa oder mit einem Harnstoff der allgemeinen Formel V

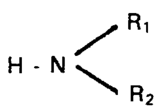

$H_2\overset{\oplus}{N}R_1R_2 \quad \overset{\ominus}{B}$

(IV) (IVa)

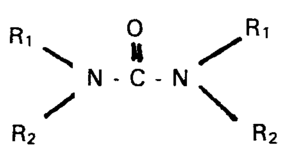

(V)

in denen $R_1$ und $R_2$ wie oben definiert sind und $B^-$ das Anion einer ein- oder mehrbasischen anorganische oder organischen Säure darstellt, in Gegenwart eines Überschusses des betreffenden Amins der allgemeinen Formel IV oder in einem polaren protischen oder aprotischen Lösungsmittel bei Temperaturen zwischen −20 und 160°C umgesetzt wird.

**Claims for the contracting states:**
BE, CH, LI, DE, FR, GB, IT, LU, NL, SE

1. New vinylogous carboxamides of general formula I

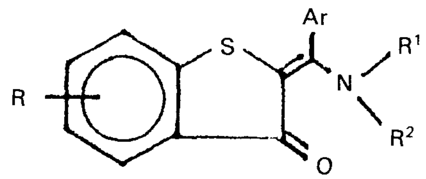

(I)

in which

Ar is a phenyl radical which may be substituted by 1 or 2 halogen atoms, alkyl groups with 1 to 3 carbon atoms, or by an amino, nitro or cyano group or by a trifluoromethyl group, or is a pyridinyl radical,

R is a hydrogen or chlorine atom or a methyl group,

$R_1$ is a hydrogen atom or a methyl group,

$R_2$ is a hydrogen atom; a straight-chained or branched alkyl radical with a maximum of 6 carbon atoms; a 2-propenyl or 2-propynyl radical; a cycloalkyl radical with from 3 to a maximum of 8 carbon atoms optionally substituted by a methyl group; or a radical $-A-R_4$, in which

A is an alkylene group with 2 or 3 carbon atoms, and

$R_4$ is a hydroxy group or a methylamino, dimethylamino, N-methyl-ethylamino, diethylamino, pyrrolidino, piperidino, hexamethyleneimino, morpholino or 4-methyl-1-piperazinyl radical.

2. New vinylogous carboxamides of general formula I according to claim 1, wherein:

Ar is a phenyl radical which may be substituted in the o-position by fluorine, chlorine, bromine, methyl or ethyl,

R is a hydrogen atom,

$R_1$ is a hydrogen atom or a methyl group and

$R_2$ is a hydrogen atom, a methyl or ethyl group or a 2-hydroxyethyl or 2-(dimethylamino)-ethyl group.

3. (E)-2-[(Amino)phenylmethylene]-benzo[b]-thiophen-3(2H)-one.

4. (E)-2-[(Methylamino)phenylmethylene]-benzo[b]thiophen-3(2H)-one.

5. (E)-2-[[(2-Hydroxyethyl)amino]phenylmethylene]-benzo[b]thiophen-3(2H)-one.

6. (E)-2-[(Amino)-(2-methylphenyl)methylene]-benzo[b]thiophen-3(2H)-one.

7. (E)-2-[(Ethylamino)phenylmethylene]-benzo-[b]thiophen-3(2H)-one.

8. A medicament containing a compound as claimed in claims 1 to 7, together with one or more inert carriers and/or diluents.

9. A medicament as claimed in claim 8 for the treatment of spastic conditions and epilepsy.

10. A process for the preparation of new vinylogous carboxamides as claimed in claims 1 to 7, characterised in that

a) a 2-acyl-benzo[b]thiophen-3-ol of general formula II

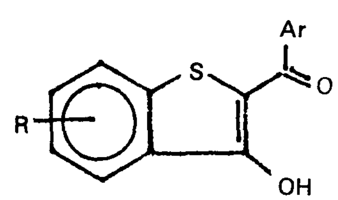

(II)

or an enol ether of general formula III

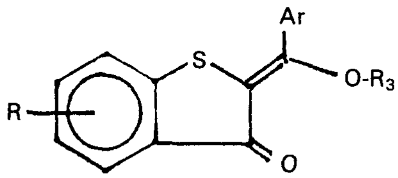

(III)

is reacted with amines of general formula IV or with ammonium salts of general formula IVa or with ureas of general formula V

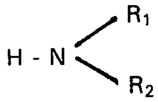

$H_2\overset{\oplus}{N}R_1R_2 \quad \overset{\ominus}{B}$

(IVa)

(IV)

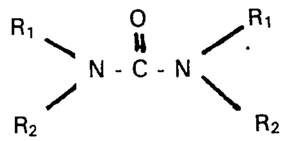

(V)

in which Ar, R, $R_1$ and $R_2$ have the meanings given in claim 1, $R_3$ is an alkyl or alkenyl group or a phenylalkyl group containing a maximum of 20 carbon atoms and optionally substituted in the benzene nucleus by halogen atoms or nitro or methyl groups and $B^-$ is the anion of a monobasic or polybasic, weak to moderately strong organic or inorganic acid, in a solvent or in an excess of the amine of general formula IV at temperatures of between −20 and +160°C or

b) for the preparation of compounds of general formula I in which $R_1$ and $R_2$ are hydrogen atoms, a 2-acyl-benzo[b]thiophen-3-ol of general formula II

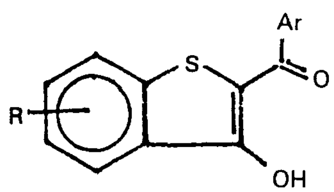

(II)

or an enol ether of general formula III

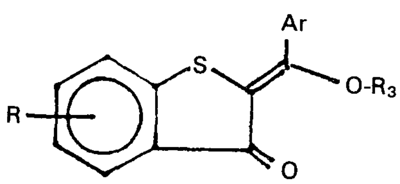

(III)

is reacted with formamide or a 2-acyl-3-chlorobenzo[b]thiophene of general formula VI

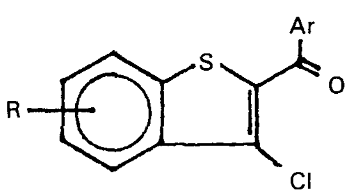

(VI)

is reacted with an ammonium salt of general formula IVb

$[NH_4]^+ \quad B^-$ (IVb)

in which formulae Ar and R are as defined hereinbefore and $B^-$ is the anion of a monobasic or polybasic weak to moderately strong organic or inorganic acid, in a solvent at temperatures of between 20 and 160°C, or

c) a 2-acyl-benzo[b]thiophene-3-ol of general formula II

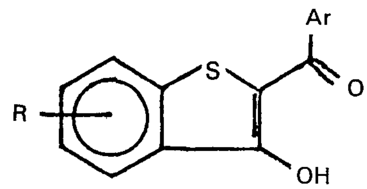

(II)

in which Ar and R are as defined hereinbefore is reacted with phosphorus pentachloride in an inert solvent at temperatures of between 0 and 40°C, the resulting product is subsequently treated with an amine of general formula IV

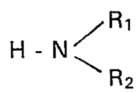

(IV)

(in which $R_1$ and $R_2$ have the meanings given hereinbefore) at between 0 and 100°C and the product formed is finally converted with aqueous dilute mineral acid at temperatures of between 30 and 100°C into the salt of the end product of general formula I from which, by treating with a base, the compound of general formula I is liberated, or

d) for the preparation of a compound of general formula I in which $R_1$ and $R_2$ represent hydrogen atoms, a compound of general formula IX

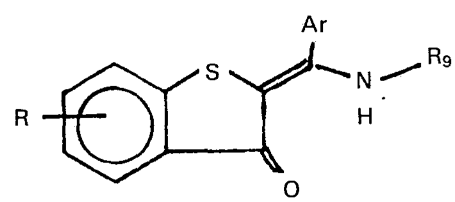

(IX)

in which Ar and R are as defined hereinbefore and $R_9$ is a hydroxy group or a radical $-NR_5R_6$ wherein $R_5$ and $R_6$ which may be identical or different are alkyl groups with 1 to 6 carbon atoms or $R_5$ is a hydrogen atom and $R_6$ is a phenyl group optionally substituted by halogen atoms or by alkyl or alkoxy groups with 1 to 3 carbon atoms, is reduced by hydrogenation in the presence of catalytically activated metals of the 8th subgroup of the Periodic Table in a solvent at temperatures of between 0 and 50°C, by treatment with nascent hydrogen and/or tin(II) chloride in a polar solvent in the presence of an acid at temperatures between 10 and 100°C, by treatment with a complex alkali metal aluminium hydride in an anhydrous ether at temperatures between −20 and +100°C, by treatment with sodium dithionite in

aqueous solution or suspension and in the presence of an alkali metal hydroxide at temperatures between 60 and 120°C, or by treatment with iron(II) hydroxide in aqueous solution or suspension at temperatures between 0 and 100°C, or

e) for the preparation of a compound of general formula I wherein $R_1$ represents a hydrogen atom and $R_2$ and R are defined as in claim 1, a carboxamide of general formula X

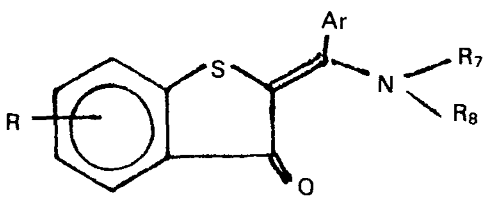

(X)

wherein

$R_7$ represents a hydrogen atom or a methyl group,

$R_8$ represents a hydrogen atom, a straight-chained or branched alkyl group with up to 6 carbon atoms, a 2-propenyl or 2-propynyl group, an optionally methyl-substituted cycloalkyl group with 3 to 8 carbon atoms or the group $-A-R_4$ and Ar, R, A and $R_4$ are as hereinbefore defined, is reacted with an amine of general formula IV, with an ammonium salt of general formula IVa or with a urea of general formula V

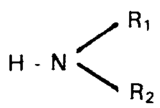

(IV)

$$H_2\overset{\oplus}{N}R_1R_2 \quad \overset{\ominus}{B}$$

(IVa)

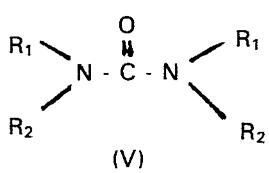

(V)

wherein $R_1$ and $R_2$ are as hereinbefore defined and $B^-$ represents the anion of a monobasic or polybasic inorganic or organic acid, in the presence of an excess of the amine of general formula IV in question or in a polar protic or aprotic solvent at temperatures of between −20 and 160°C.

**Claim for the contracting state:** AT

Process for the preparatin of new vinylogous carboxamides of general formula I

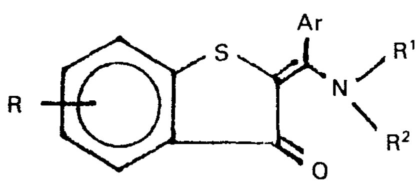

(I)

wherein

Ar is a phenyl radical which may be substituted by 1 or 2 halogen atoms, alkyl groups with up to 3 carbon atoms, or by an amino, nitro or cyano group or by a trifluoromethyl group, or is a pyridinyl radical,

R is a hydrogen or chlorine atom or a methyl group,

$R_1$ is a hydrogen atom or a methyl group,

$R_2$ is a hydrogen atom; a straight-chained or branched alkyl radical with a maximum of 6 carbon atoms; a 2-propenyl or 2-propynyl radical; a cycloalkyl radical with from 3 to a maximum of 8 carbon atoms optionally substituted by a methyl group; or a radical $-A-R_4$, in which

A is an alkylene group with 2 or 3 carbon atoms, and

$R_4$ is a hydroxy group or a methylamino, dimethylamino, N-methyl-ethylamino, diethylamino, pyrrolidino, piperidino, hexamethyleneimino, morpholino or 4-methyl-1-piperazinyl radical, characterised in that

a) a 2-acyl-benzo[b]thiophen-3-ol of general formula II

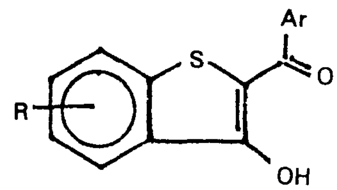

(II)

or an enol ether of general formula III

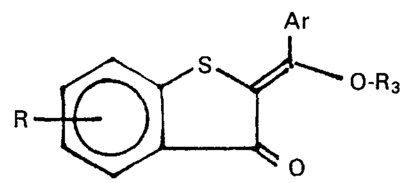

(III)

is reacted with amines of general formula IV or with ammonium salts of general formula IVa or with ureas of general formula V

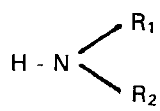

(IV)

$$H_2\overset{\oplus}{N}R_1R_2 \quad \overset{\ominus}{B}$$

(IVa)

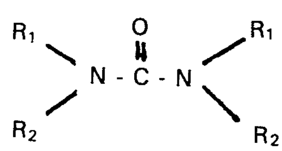

(V)

in which Ar, R, $R_1$ and $R_2$ have the meanings given hereinbefore, $R_3$ is an alkyl or alkenyl group or a phenylalkyl group containing a maximum of 20 carbon atoms and optionally substituted in the benzene nucleus by halogen atoms or nitro or methyl groups and $B^-$ is the anion of a monobasic or polybasic, weak to moderately strong organic or inorganic acid, in a solvent or in an excess of the amine of general

formula IV at temperatures of between −20 and +160°C or

b) for the preparation of compounds of general formula I in which $R_1$ and $R_2$ are hydrogen atoms, a 2-acyl-benzo[b]thiophen-3-ol of general formula II

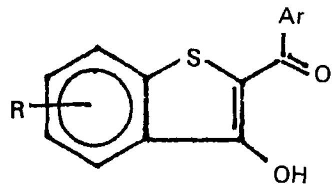

(II)

or an enol ether of general formula III

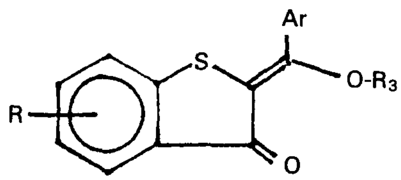

(III)

is reacted with formamide or a 2-acyl-3-chloro-benzo[b]thiophene of general formula VI

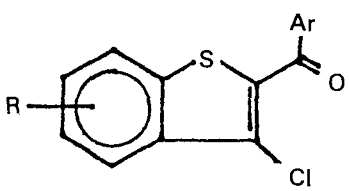

(VI)

is reacted with an ammonium salt of general formula IVb

$[NH_4]^{+1}$ $B^-$ (IVb)

in which formulae Ar and R are as defined hereinbefore and $B^-$ is the anion of a monobasic or polybasic weak to moderately strong organic or inorganic acid, in a solvent at temperatures of between 20 and 160°C, or

c) a 2-acyl-benzo[b]thiophen-3-ol of general formula II

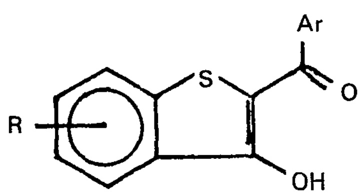

(II)

in which Ar and R are as defined hereinbefore is reacted with phosphorus pentachloride in an inert solvent at temperatures of between 0 and 40°C, the resulting product is subsequently treated with an amine of formula IV

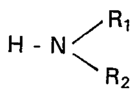

(IV)

(in which $R_1$ and $R_2$ have the meanings given hereinbefore) at between 0 and 100°C and the product formed is finally converted with aqueous dilute mineral acid at temperatures of between 30 and 100°C into the salt of the end product of general formula I from which, by treating with a base, the compound of general formula I is liberated, or

d) for the preparation of a compound of general formula I in which $R_1$ and $R_2$ represent hydrogen atoms, a compound of general formula IX

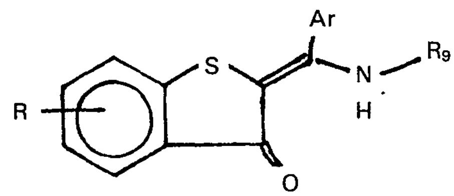

(IX)

in which Ar and R are as defined hereinbefore and $R_9$ is a hydroxy group or a radical $-NR_5R_6$ wherein $R_5$ and $R_6$ which may be identical or different are alkyl groups with 1 to 6 carbon atoms or $R_5$ is a hydrogen atom and $R_6$ is a phenyl group optionally substituted by halogen atoms or by alkyl or alkoxy groups with 1 to 3 carbon atoms, is reduced by hydrogenation in the presence of catalytically activated metals of the 8th subgroup of the Periodic Table in a solvent at temperatures of between 0 and 50°C, by treatment with nascent hydrogen and/or tin(II) chloride in a polar solvent in the presence of an acid at temperatures between 10 and 100°C, by treatment with a complex alkali metal aluminium hydride in an anhydrous ether at temperatures between −20 and +100°C, by treatment with sodium dithionite in aqueous solution or suspension and in the presence of an alkali metal hydroxide at temperatures between 60 and 120°C, or by treatment with iron(II) hydroxide in aqueous solution or suspension at temperatures between 0 and 100°C, or

e) for the preparation of a compound of general formula I wherein $R_1$ represents a hydrogen atom and $R_2$ and R are defined as in claim 1, a carboxamide of general formula X

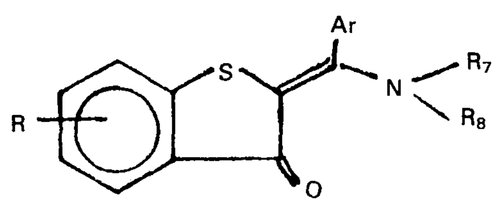

(X)

wherein

$R_7$ represents a hydrogen atom or a methyl group,

$R_8$ represents a hydrogen atom, a straight-chained or branched alkyl group with up to 6 carbon atoms, a 2-propenyl or 2-propynyl group, an optionally methyl-substituted cycloalkyl group with 3 to 8 carbon atoms or the group $-A-R_4$ and Ar, R, A and $R_4$ are as hereinbefore defined, is reacted with an amine of general formula IV, with an ammonium salt of general formula IVa or with a urea of general formula V

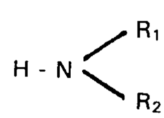

(IV)

$H_2\overset{\oplus}{N}R_1R_2$ $\overset{\ominus}{B}$

(IVa)

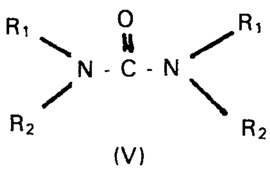

(V)

wherein $R_1$ and $R_2$ are as hereinbefore defined and $B^-$ represents the anion of a monobasic or polybasic inorganic or organic acid, in the presence of an excess of the amine of general formula IV in question or in a polar protic or aprotic solvent at temperatures of between −20 and 160°C.

**Revendications pour les Etats contractants:**
BE, CH, LI, DE, FR, GB, IT, LU, NL, SE

1. Nouveaux carboxamides vinylogues de formule générale I

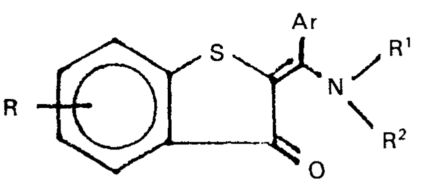

(I)

dans laquelle

Ar représente un radical phényle qui peut être substitué par 1 ou 2 atomes d'halogène, des groupes alcoyle avec 1 à 3 atomes de carbone, le groupe amino, nitro ou cyano ou un radical trifluorométhyle, ou représente un radical pyridinyle,

R représente un atome d'hydrogène ou de chlore ou le groupe méthyle,

$R_1$ représente un atome d'hydrogène ou le groupe méthyle,

$R_2$ représente un atome d'hydrogène, un radical alcoyle rectiligne ou ramifié avec au plus 6 atomes de carbone, un radical 2-propényle ou 2-propynyle, un radical cycloalcoyle avec 3 jusqu'à 8 atomes de carbone au maximum, éventuellement méthylsubstitué ou le radical -A-$R_4$,

A représentant un groupe alcoylène avec 2 ou 3 atomes de carbone, et

$R_4$ représentant le groupe hydroxy, le radical méthylamino, diméthylamino, N-méthyl-éthylamino, diéthylamino, pyrrolidino, pipéridino, hexaméthylèneimino, morpholino ou 4-méthyl-1-pipérazinyle.

2. Nouveaux carboxamides vinylogues de formule générale I selon la revendication 1, dans laquelle

Ar représente un radical phényle qui peut être substitué en position o par du fluor, du chlore, du brome, méthyle ou éthyle,

R représente un atome d'hydrogène,

$R_1$ représente un atome d'hydrogène ou le groupe méthyle,

$R_2$ représente un atome d'hydrogène, le groupe méthyle, éthyle, 2-hydroxy-éthyle ou 2-(diméthylamino)éthyle.

3. La (E)-2-[(amino)phénylméthylène]-benzo[b]-thiophène-3(2H)-one.

4. La (E)-2-[(méthylamino)phénylméthylène]-benzo[b]thiophène-3(2H)-one.

5. La (E)-2-[[(2-hydroxyéthyl)amino]phénylméthylène]-benzo[b]thiophène-3(2H)-one.

6. La (E)-2-[(amino)-(2-méthylphényl)méthylène]-benzo[b]thiophène-3(2H)-one.

7. La (E)-2-[(éthylamino)phénylméthylène]-benzo[b]thiophène-3(2H)-one.

8. Médicament contenant un composé selon les revendications 1 à 7 conjointement à un ou plusieurs excipients et/ou diluants inertes.

9. Médicament selon la revendication 8 pour le traitement des états spasmodiques et de l'épilepsie.

10. Procédé pour la préparation de nouveaux carboxamides vinylogues selon les revendications 1 à 7, caractérisé en ce que

a) on fait réagir un 2-acyl-benzo[b]thiophène,3-ol de formule générale II

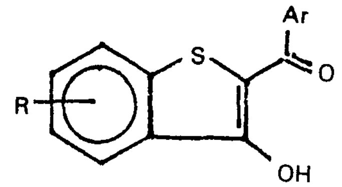

(II)

ou un énoléther de formule générale III

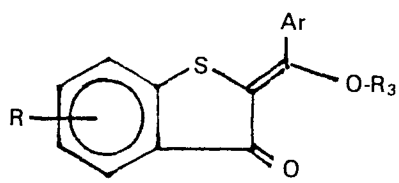

(III)

avec des amines de formule générale IV ou avec des sels d'ammonium de formule générale IVa ou avec des urées de formule générale V

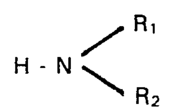

(IV)

$H_2\overset{\oplus}{N}R_1R_2 \quad \overset{\ominus}{B}$

(IVa)

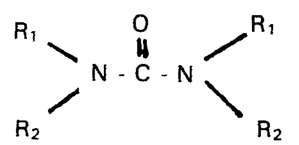

(V)

dans lesquelles Ar, R, $R_1$ et $R_2$ ont les significations indiquées dans la revendication 1, $R_3$ représente un groupe alcoyle, alcènyle ou phénylalcoyle avec au maximum 20 atomes de carbone, éventuellement substitué dans le noyau benzène par des atomes d'halogène, des groupes nitro ou méthyle et $B^-$ représente l'anion d'un acide minéral ou organique mono ou plurifonctionnel, faible à moyennement fort, dans un solvant ou dans un excès de l'amine de formule générale IV à des températures entre −20 et +160°C ou

b) pour la préparation de composés de formule générale I dans laquelle $R_1$ et $R_2$ représentent des atomes d'hydrogène, on fait réagir un 2-acyl-benzo[b]-thiophène-3-ol de formule générale II

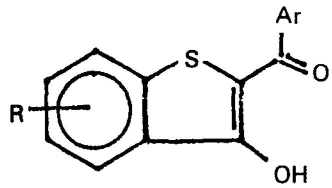

(II)

ou un énoléther de formule générale III

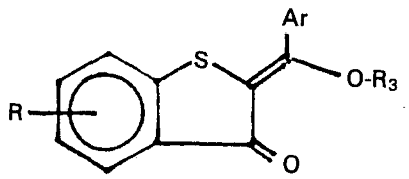

(III)

avec du formamide ou un 2-acyl-3-chloro-benzo[b]thiophène de formule générale VI

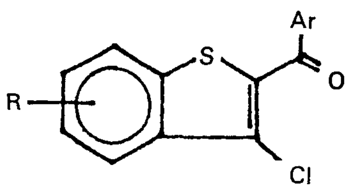

(VI)

avec un sel d'ammonium de formule générale IVb

$[NH_4]^+ \quad B^-$ (IVb)

dans ces formules Ar et R étant définis comme plus haut et $B^-$ représentant l'anion d'un acide minéral ou organique mono ou plurifonctionnel faible à moyennement fort, dans un solvant à des températures entre 20 et 160°C, ou

c) on fait réagir un 2-acyl-benzo[b]thiophène-3-ol de formule générale II

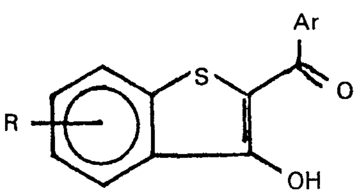

(II)

dans laquelle Ar et R sont définis comme indiqué au début, avec du pentachlorure de phosphore dans un solvant inerte à des températures entre 0 et 40°C, on traite ensuite entre 0 et 100°C le produit alors formé avec une amine de formule générale IV

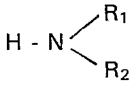

(IV)

dans laquelle $R_1$ et $R_2$ possèdent les significations indiquées plus haut et on transforme ensuite le produit se formant au moyen d'acide minéral aqueux, dilué, à des températures entre 30 et 100°C en le sel du produit final de formule générale I à partir duquel on libère par traitement avec une base le composé de formule générale I, ou

d) pour la préparation d'un composé de formule générale I dans laquelle $R_1$ et $R_2$ représentent des atomes d'hydrogène, on réduit un composé de formule générale IX

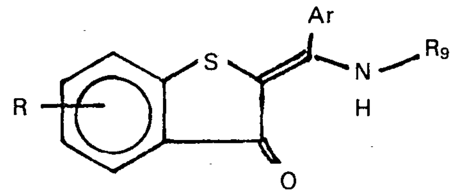

(IX)

dans laquelle Ar et R sont définis comme plus haut et $R_9$ représente le groupe hydroxy ou le radical $-NR_5R_6$, où $R_5$ et $R_6$ représentent des groupes alcoyle avec 1 à 6 atomes de carbone soit identiques soit différents l'un de l'autre ou bien $R_5$ représente un atome d'hydrogène et $R_6$ représente un groupe phényle éventuellement substitué par des atomes d'halogène ou des groupes alcoyle ou alxocy avec 1 à 3 atomes de carbone, par hydrogènation en présence de métaux à activité catalytique du 8e sous-groupe de la classification périodique des éléments dans des solvants à des températures entre 0 et 50°C, par traitement au moyen d'hydrogène naissant et/ou de chlorure d'étain(II) dans des solvants polaires en présence d'un acide à des températures entre 10 et 100°C, par traitement au moyen d'hydrures d'aluminium et de métal alcalin complexes dans des éthers anhydres à des températures entre —20 et +100°C, par traitement au moyen de dithionite de sodium en solution ou suspension aqueuse et en présence d'hydroxyde alcalin à des températures entre 60 et 120°C ou par traitement au moyen d'hydroxyde de fer(II) en solution ou suspension aqueuse à des températures entre 0 et 100°C;

e) pour la préparation d'un composé de formule générale I dans laquelle $R_1$ représente un atome d'hydrogène et $R_2$ et R ont la signification indiquée dans la revendication 1, on fait réagir un carboxamide de formule générale X

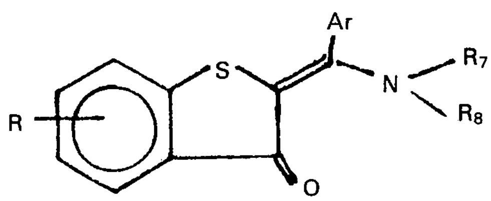

(X)

dans laquelle

$R_7$ représente un atome d'hydrogène ou le groupe méthyle,

$R_8$ représente un atome d'hydrogène, un radical alcoyle rectiligne ou ramifié avec jusqu'à 6 atomes de carbone, un radical 2-propényle ou 2-propynyle, un radical cycloalcoyle avec 3 à 8 atomes de carbone, éventuellement méthylsubstitué ou le radical $-A-R_4$ et Ar, R, A et $R_4$ sont définis comme plus haut, avec une amine de formule générale IV, avec un sel d'ammonium de formule générale IVa ou avec une urée de formule générale V

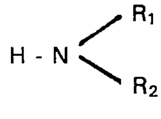

(IV)

$H_2\overset{\oplus}{N}R_1R_2 \quad \overset{\ominus}{B}$

(IVa)

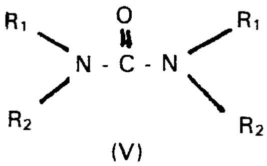

(V)

dans lesquelles $R_1$ et $R_2$ sont définis comme plus haut et $B^-$ représente l'anion d'un acide minéral ou organique mono ou plurifonctionnel, en présence d'un excès de l'amine correspondante de formule générale IV ou dans un solvant polaire protique ou aprotique à des températures entre $-20$ et 160°C.

**Revendication pour l'Etat contractant:** AT

Procédé pour la préparation de nouveaux carboxamides vinylogues de formule générale I

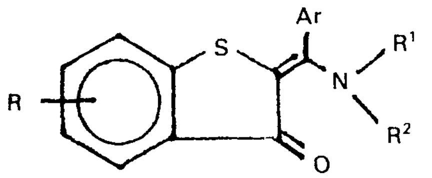

(I)

dans laquelle

Ar représente un radical phényle qui peut être substitué par 1 ou 2 atomes d'halogène, des groupes alcoyle avec jusqu'à 3 atomes de carbone, le groupe amino, nitro ou cyano ou le groupe trifluorométhyle, ou représente un radical pyridinyle,

R représente un atome d'hydrogène ou de chlore ou le groupe méthyle,

$R_1$ représente un atome d'hydrogène ou le groupe méthyle,

$R_2$ représente un atome d'hydrogène, un radical alcoyle rectiligne ou ramifié avec au plus 6 atomes de carbone, un radical 2-propényle ou 2-propynyle, un radical cycloalcoyle avec 3 à 8 atomes de carbone au maximum, éventuellement méthylsubstitué ou le radical -A-$R_4$,

A représentant un groupe alcoylène avec 2 ou 3 atomes de carbone,

$R_4$ représentant le groupe hydroxy, le radical méthylamino, diméthylamino, N-méthyl-éthylamino, diéthylamino, pyrrolidino, pipéridino, hexaméthylèneimino, morpholino ou 4-méthyl-1-pipérazinyle, caractérisé en ce que

a) on fait réagir un 2-acyl-benzo[b]thiophène-3-ol de formule générale II

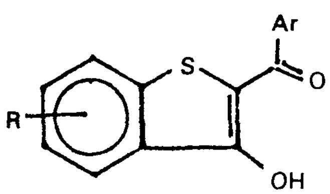

(II)

ou un énoléther de formule générale III

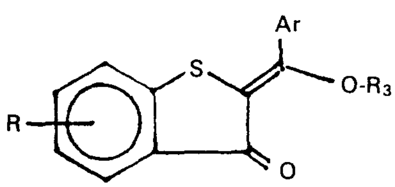

(III)

avec des amines de formule générale IV, avec des sels d'ammonium de formule générale IVa ou avec des urées de formule générale V

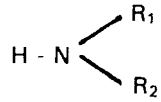

(IV)

$H_2NR_1R_2^{\oplus}$ $B^{\ominus}$

(IVa)

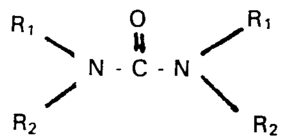

(V)

dans lesquelles Ar, R, $R_1$ et $R_2$ ont les significations indiquées au début, $R_3$ représente un groupe alcoyle, alcènyle ou phénylalcoyle avec au maximum 20 atomes de carbone, éventuellement substitué dans le noyau benzène par des atomes d'halogène, des groupes nitro ou méthyle et $B^-$ représente l'anion d'un acide minéral ou organique mono ou plurifonctionnel, faible à moyennement fort, dans un solvant ou dans un excès de l'amine de formule générale IV à des températures entre $-20$ et $+160$°C ou

b) pour la préparation de composés de formule générale I dans laquelle $R_1$ et $R_2$ représentent des atomes d'hydrogène, on fait réagir un 2-acyl-benzo[b]-thiophène-3-ol de formule générale II

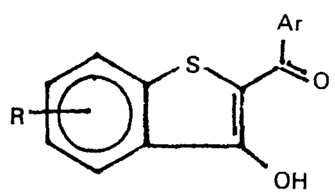

(II)

ou un énoléther de formule générale III

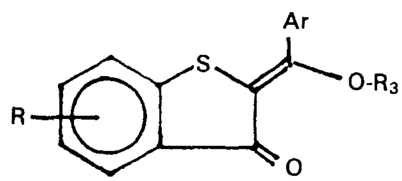

(III)

avec du formamide ou un 2-acyl-3-chloro-benzo[b]thiophène de formule générale VI

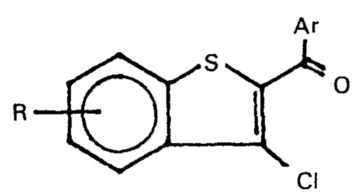

(VI)

avec un sel d'ammonium de formule générale IVb

$[NH_4]^+$ $B^-$ (IVb)

dans ces formules Ar et R étant définis comme plus haut et $B^-$ représentant l'anion d'un acide minéral ou organique mono ou plurifonctionnel faible à moyennement fort, dans un solvant à des températures entre 20 et 160°C, ou

c) on fait réagir un 2-acyl-benzo[b]thiophène-3-ol de formule générale II

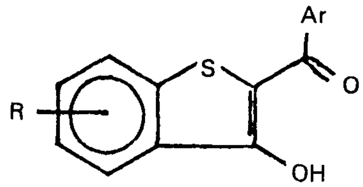

(II)

dans laquelle Ar et R sont définis comme indiqué au début, avec du pentachlorure de phosphore dans un solvant inerte à des températures entre 0 et 40°C, on traite ensuite entre 0 et 100°C le produit ainsi formé avec une amine de formule générale IV

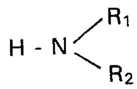

(IV)

dans laquelle $R_1$ et $R_2$ possèdent les significations indiquées plus haut et on transforme ensuite le produit se formant au moyen d'acide minéral aqueux, dilué, à des températures entre 30 et 100°C en le sel du produit final de formule générale I, à partir duquel on libère le composé de formule générale I par traitement par une base, ou

d) pour la préparation d'un composé de formule générale I dans laquelle $R_1$ et $R_2$ représentent des atomes d'hydrogène, on réduit un composé de formule générale IX

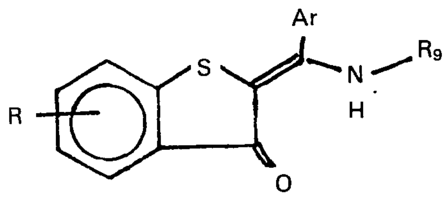

(IX)

dans laquelle Ar et R sont définis comme plus haut et $R_9$ représente le groupe hydroxy ou le radical $-NR_5R_6$, dans lequel $R_5$ et $R_6$ représentent des groupes alcoyle avec 1 à 6 atomes de carbone soit identiques soit différents l'un de l'autre ou bien $R_5$ représente un atome d'hydrogène et $R_6$ représente un groupe phényle éventuellement substitué par des atomes d'halogène ou des groupes alcoyle ou alcoxy avec 1 à 3 atomes de carbone, par hydrogènation en présence de métaux à activité catalytique du 8e sous-groupe de la classification périodique des éléments, dans des solvants à des températures entre 0 et 50°C, par traitement au moyen d'hydrogène naissant et/ou chlorure d'étain(II) dans des solvants polaires en présence d'un acide à des températures entre 10 et 100°C, par traitement au moyen d'hydrures d'aluminium et de métal alcalin complexes dans des éthers anhydres à des températures entre −20 et +100°C, par traitement par le dithionite de sodium en solution ou suspension aqueuse et en présence d'hydroxyde alcalin à des températures entre 60 et 120°C ou par traitement au moyen d'hydroxyde de fer(II) en solution ou suspension aqueuse à des températures entre 0 et 100°C;

e) pour la préparation d'un composé de formule générale I dans laquelle $R_1$ représente un atome d'hydrogène et $R_2$ et R possèdent la signification indiquée plus haut, on fait réagir un carboxamide de formule générale X

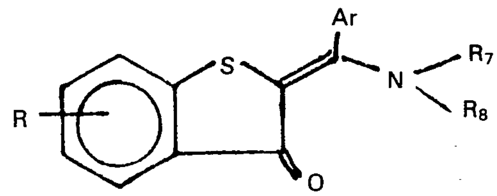

(X)

dans laquelle

$R_7$ représente un atome d'hydrogène ou le groupe méthyle,

$R_8$ représente un atome d'hydrogène, un radical alcoyle rectiligne ou ramifié avec jusqu'à 6 atomes de carbone, un radical 2-propényle ou 2-propynyle, un radical cycloalcoyle avec 3 à 8 atomes de carbone éventuellement méthylsubstitué ou le radical $-A-R_4$ et Ar, R, A et $R_4$ sont définis comme plus haut, avec une amine de formule générale IV ou avec un sel d'ammonium de formule générale IVa ou avec une urée de formule générale V

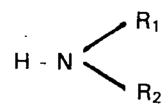

(IV)

$H_2\overset{\oplus}{N}R_1R_2 \quad \overset{\ominus}{B}$

(IVa)

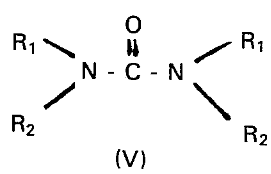

(V)

dans lesquelle $R_1$ et $R_2$ sont définis comme plus haut et $B^-$ représente l'anion d'un acide minéral ou organique mono ou plurifonctionnel, en présence d'un excès de l'amine correspondante de formule générale IV ou dans un solvant polaire protique ou aprotique à des températures entre −20 et 160°C.